(19) Europäisches Patentamt
European Patent Office
Office européen des brevets

(11) Veröffentlichungsnummer: **0 017 760**
**B1**

(12) # EUROPÄISCHE PATENTSCHRIFT

(45) Veröffentlichungstag der Patentschrift: **11.05.83**

(21) Anmeldenummer: **80101310.3**

(22) Anmeldetag: **13.03.80**

(51) Int. Cl.³: **C 07 C103/52**, A 61 K 37/02

(54) **Somatostatin-analoge Peptide, ihre Herstellung und Verwendung, sie enthaltende pharmazeutische Präparate und deren Herstellung.**

(30) Priorität: **16.03.79 CH 2506/79**

(43) Veröffentlichungstag der Anmeldung: **29.10.80 Patentblatt 80/22**

(45) Bekanntmachung des Hinweises auf die Patenterteilung: **11.05.83 Patentblatt 83/19**

(84) Benannte Vertragsstaaten: **AT BE CH DE FR GB IT LU NL SE**

(56) Entgegenhaltungen:
**DE A 2 634 416**
**DE A 2 635 558**
**US A 4 098 782**
**US A 4 122 077**

(73) Patentinhaber: **CIBA-GEIGY AG**
**Patentabteilung Postfach**
**CH-4002 Basel (CH)**

(72) Erfinder: **Kamber, Bruno, Dr.**
**Sonnenweg 9**
**CH-4144 Arlesheim (CH)**
Erfinder: **Rink, Hans**
**Rebenstrasse 10**
**CH-4125 Riehen (CH)**
Erfinder: **Sieber, Peter**
**Bachmattweg 10**
**CH-4153 Reinach (CH)**

(74) Vertreter: **Zumstein, Fritz sen., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**

**EP 0 017 760 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Somatostatin-analoge Peptide, ihre Herstellung und Verwendung, sie enthaltende pharmazeutische Präparate und deren Herstellung

Die Erfindung betrifft neue Peptide vom Somatostatin-Typ und ihre Herstellungsverfahren, sowie pharmazeutische Präparate enthaltend diese Verbindungen, und die Verwendung dieser Verbindungen bzw. Präparate zu therapeutischen Zwecken. Die Erfindung betrifft insbesondere Peptide, welche im Vergleich zu Somatostatin eine um die ersten 2 N-terminalen Aminosäuren kürzere und durch einige Ersatzglieder modifizierte Aminosäuresequenz aufweist.

Die erfindungsgemässen Somatostatin-analogen Peptide umfassen modifizierte Des-[Ala[1]-Gly[2]]-desamino-Cys[3]-somatostatine der allgemeinen Formel

Bmp-Lys-X-Phe-Phe-trp-Lys-Thr-Phe-Thr-Y-Cys-OH
3 4 5 6 7 8 9 10 11 12 13 14

worin Bmp für den Desaminocystein-Rest (d. h. den $\beta$-Mercaptopropionyl-Rest), X für Asn oder His, trp für D-Trp, welches im Benzolring durch ein Halogenatom substituiert sein kann, und Y für den Rest einer sekundären $\alpha$-Aminosäure mit höchstens 8 Kohlenstoffatomen steht, und die entsprechenden Peptidamide, sowie Säureadditionssalze und Komplexe aller dieser Verbindungen.

Das im Benzolring des D-Tryptophan[8]-Rests gegebenenfalls vorhandene Halogenatom ist insbesondere ein Chlor- oder Fluoratom, das sich vorzugsweise in der 5-Stellung befindet; besonders zu nennen ist der 5-Fluor-D-tryptophyl[8]-Rest.

Die als Y bezeichnete sekundäre $\alpha$-Aminosäure ist eine $\alpha$-Niederalkylaminoniederalkylcarbonsäure, in welcher die beiden Niederalkylreste durch eine C-C-Bindung, ein Sauerstoffatom, ein Schwefel(II)-atom oder ein gegebenenfalls niederalkyliertes Stickstoffatom zusammen verbunden sein können, wobei jeder einzelne der Niederalkylreste höchstens 6 Kohlenstoffatome und alle zusammen höchstens 7 Kohlenstoffatome enthalten. Der Niederalkylrest, welcher dem Kohlenstoffgerüst der Carbonsäure zugrunde liegt, weist vorzugsweise mehr als ein C-Atom auf und ist insbesondere ein solcher, der in den natürlichen Aminosäuren vorkommt, wie Butyl, Isobutyl, Pentyl und insbesondere Aethyl und Isopentyl. Das Niederalkyl, das als Substituent der Aminogruppe oder der Stickstoffbrücke vorkommt, ist vorzugsweise Methyl. Die C-C-Bindung, welche die beiden Niederalkylreste gegebenenfalls verbindet, ist vorzugsweise eine Einfachbindung. Die $\alpha$-Aminogruppe befindet sich vorzugsweise in einer solchen sterischen Konfiguration, die den natürlichen Aminosäuren, d. h. den L-Aminosäuren, entspricht.

In der Bedeutung von Y sind insbesondere die Reste solcher sekundären $\alpha$-Aminosäuren bevorzugt, die als in der Natur vorkommende Aminosäuren bekannt sind, wie vor allem L-Prolin der Formel

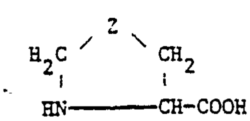

worin Z die Methylengruppe ist, oder ihnen unmittelbar strukturell analog sind, wie einerseits 4-Oxa- und insbesondere 4-Thiaprolin der obigen Formel, worin Z Sauerstoff oder Schwefel ist, und andererseits eine N-niederalkylierte, insbesondere N-methylierte, aliphatische Aminosäure, vor allem N-Methyl-L-leucin.

Die bevorzugten erfindungsgemässen Somatostatin-Analogen sind solche Verbindungen der Formel I, worin X für Asn, trp für D-Trp oder 5-Fluor-D-Trp und Y für Pro, Tpo (d. h. den Rest des oben charakterisierten 4-Thiaprolins) oder MeLeu (d. h. N-Methyl-Leu) steht. Vor allem sind zu nennen: Desamino-Cys[3]-D-Trp[8]-Pro[13]-somatostatin(3-14) [Formel I; X = Asn, trp = D-Trp und Y = Pro]; Desamino-Cys[3]-[(5-F)-D-Trp[8]]-Pro[13]-somatostatin(3-14) [Formel I; X = Asn, trp = 5-Fluor-D-Trp, Y = Pro]; Desamino-Cys[3]-D-Trp[8]-Tpo[13]-somatostatin(3-14) [Formel I; X = Asn, trp = D-Trp, Y = 4-Thia-Pro] und Desamino-Cys[3]-D-Trp[8]-MeLeu[13]-somatostatin(3-14) [Formel I, X = Asn, trp = D-Trp, Y = N-Methyl-Leu]. Alle diese bevorzugten Somatostatin-Analogen können auch in Form von Säureadditionssalzen oder Komplexen vorliegen.

Als Säureadditionssalze kommen besonders physiologisch verträgliche Salze mit üblichen therapeutisch anwendbaren Säuren in Betracht; von den anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, wie die Benzol- oder p-Toluol-Sulfonsäure, oder Niederalkansulfonsäuren, wie Methansulfonsäure, ferner auch Carbonsäuren, wie Essigsäure, Milchsäure, Palmitin- und Stearinsäure, Aepfelsäure, Weinsäuren, Ascorbinsäure und Zitronensäure.

Unter Komplexen sind die in ihrer Struktur noch nicht völlig abgeklärten Verbindungen zu verstehen,

die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z. B. anorganische Verbindungen, die sich von Metallen wie Calcium, Magnesium, Aluminium, Cobalt und insbesondere von Zink ableiten, vor allem schwerlösliche Salze, wie Phosphate, Pyrophosphate und Polyphosphate, sowie Hydroxide dieser Metalle, ferner Alkalimetall-polyphosphate, z. B. Calgon N®, Calgon 322®, oder Calgon 188®. Organische Stoffe, die eine Verlängerung der Wirkung hervorrufen, sind beispielsweise nicht antigene Gelatine-Arten, z. B. Polyoxy-gelatine, Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z. B. Protamin oder Polyglutaminsäure.

Wenn nicht anders angegeben, beziehen sich die Kurzbezeichnungen der Aminosäurereste auf Reste der α-Aminosäuren der L-Reihe, welche in der Natur vorkommen.

Wenn nicht anders angegeben, bezeichnet der Ausdruck « nieder », wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemässen Peptide weisen eine physiologische Wirkung auf, die im Grundcharakter der Wirkung des Somatostatins ähnlich ist. Sie können deshalb in ähnlichen therapeutischen Indikationen wie dieses, z. B. insbesondere zur Behandlung von Funktionsstörungen, in denen die Sekretion des Wachstumshormons oder Glucagons übernormal hoch ist, wie bei Acromegalie oder Diabetes, mit Vorteil angewendet werden. Indem sie überdies auch Blutverluste im gastrointestinalen Trakt hemmen, können sie auch in diesem Indikationsbereich mit Erfolg eingesetzt werden.

Somatostatin, ein cyclisches Tetradecapeptid der Formel

H-Ala-Gly-Cys-Lys-Asn-Phe-Phe-Trp-Lys-Thr-Phe-Thr-Ser-Cys-OH
1 2 3 4 5 6 7 8 9 10 11 12 13 14

[Science *179*, 77 (1973)] hemmt bekanntlich die hypophysengesteuerte Sekretion des Wachstumshormons (Somatotropin). Ausserdem hemmt es die sekretorische Aktivität des endocrinen Pancreas, wie die Sekretion von Insulin und Glucagon. Diese wertvollen Eigenschaften können beim Somatostatin selber nicht zur vollen praktischen Anwendung gelangen, da oft die hemmende Wirkung auf nur eine der beiden Drüsen erwünscht ist, wogegen die andere nach Möglichkeit unterdrückt werden sollte. In den meisten Fällen ist die Hemmung der hypophysären Sekretion (d. h. die der Wachstumshormon-Ausschüttung) eher als Nachteil bei den häufigsten therapeutischen Indikationen empfunden. Aus diesem Grunde wurde nach analogen Strukturen gesucht, bei welchen man durch geeignete Modifizierung der Grundsequenz, insbesondere durch Auslassen einzelner ursprünglicher Aminosäuren und/oder deren Austausch gegen andere, oft auch « unnatürliche » Aminosäuren, eine Potenzierung bzw. Verlängerung des allgemeinen Wirkungseffektes und vor allem eine Dissoziation der Hemmwirkungen erreichen kann. — Insbesondere wurden die Aminosäuren der Seitenkette (Stellungen 1 und 2) weggelassen und/oder L-$Trp^8$ gegen D-$Trp^8$ ausgetauscht, vgl. DE-A1-2 634 416 (Salk) ; zusätzlich zu diesen Modifikationen wurde noch ein oder beide Schwefelatome der Cystin-Brücke oxidiert, gegen $CH_2$ ausgetauscht oder weggelassen, vgl. DE-A1-2 635 558 (Merck), oder weitere Aminosäuren der D-Reihe in die Stellungen 4 und/oder 5 eingeführt, vgl. US-A-4 122 077 (Sarantakis), oder aber $Asn^5$ gegen $His^5$ ausgetauscht, vgl. US-A-4 098 782 (Sarantakis). Nur in diesem letzten Fall wurde eine Dissoziation der Wirkung erwähnt, wobei die Hemmung der Sekretion von Wachstumshormon und Glucagon, nicht aber von Insulin, erfolgte.

Ueberraschenderweise wurde nun festgestellt, dass man in einem verkürzten Molekül des Somatostatins den ursprünglichen Serin-Rest durch Reste anderer, sogar « unnatürlicher » Aminosäuren, welche einzeln keinen nennenswerten Einfluss haben, so ersetzen kann, dass dabei ein Teil der physiologischen Aktivität nicht nur erhalten bleibt, sondern sogar gesteigert wird. Als besonders vorteilhaft erwiesen sich dabei überraschenderweise α-Aminosäuren der L-Reihe mit einer sekundären Aminogruppe, obwohl ein günstiges Resultat unwahrscheinlich war, da eine analoge Umwandlung der primären Aminogruppe in eine sekundäre bei Aminosäuren in der 7- und 11-Stellung von Somatostatin zu einer wesentlichen Senkung, wenn nicht sogar zur praktischen Aufhebung jeder pharmakologischen Wirksamkeit führte, vgl. J. Rivier et al : Peptides 1976 (Proceedings of the Fourteenth European Peptide Symposium ; Wepion, Belgium ; April 11-17, 1976), Seiten 439, 440, 442 und 443.

Wie nun gefunden wurde, weisen die erfindungsgemässen Verbindungen eine gegenüber Somatostatin erhöhte Hemmung der Insulin- und Glucagon-Sekretion bei einer gleichbleibenden oder sogar gesenkten hemmenden Wirkung auf die hypophysäre Sekretion des Wachstumshormons auf.

Die erfindungsgemässen Verbindungen können nach an sich bekannten Methoden hergestellt werden. Insbesondere werden sie erhalten, indem man in einer Verbindung der allgemeinen Formel

Bmp(C)-Lys(A)-X-Phe-Phe-trp-Lys(A')-Thr(B)-Phe-Thr(B')-Y-Cys(C)-D (II)

worin X, trp und Y die obgenannten Bedeutungen haben und A und A' unabhängig voneinander je eine ε-Amino-Schutzgruppe oder Wasserstoff, B und B' unabhängig voneinander je eine Hydroxyl-Schutzgruppe oder Wasserstoff, C und C' unabhängig voneinander je eine Mercapto-Schutzgruppe oder Wasserstoff und D eine Carboxyl-Schutzgruppe, die Aminogruppe $NH_2$ oder Hydroxyl bedeuten, in beliebiger Reihenfolge a) vorhandene Schutzgruppen abspaltet und b) zwischen den Mercaptogruppen beider endständigen Säuren gegebenenfalls unter gleichzeitiger Abspaltung vorhandener Mercapto-Schutzgruppen die Disulfid-Brücke bildet und, wenn erwünscht, einen als Säureadditionssalz resultierenden Endstoff in die entsprechende freie Base oder einen als Base resultierenden Endstoff in ein Säureadditionssalz davon umwandelt und/oder, wenn erwünscht, einen Endstoff in Form eines Komplexes isoliert.

Eine Variante des erfindungsgemässen Verfahrens besteht z. B. darin, dass man im Ausgangsstoff der oben definierten Formel II alle vorhandenen Schutzgruppen, bis auf C oder C', abspaltet, was bei geeigneter Wahl von Schutzgruppen mit Vorteil in einer einzigen Operation verwirklicht werden kann, und nachher die als Zwischenprodukt vorliegende Verbindung der Formel II, worin je A, A', B, und B' Wasserstoff, D Hydroxyl oder die Aminogruppe $NH_2$, einer der Symbole C und C' Wasserstoff und das andere eine n-Alkylthiogruppe oder der Rest H-Cys-OH ist, in an sich bekannter Weise, z. B. gemäss U.S. Patent 3 929 758 bzw. der Deutschen Offenlegungsschrift Nr. 26 47843, in einer im wesentlichen sauerstoffreien Lösung bei pH 5-10 zur Cyclisierung hält. Als Schutzgruppen A, A', B und B' sind in diesem Fall insbesondere acidolytisch abspaltbare Gruppen, z. B. die weiter unten angegebenen, besonders geeignet, als eine der Mercaptoschutzgruppen C und C' ist ebenso eine acidolytisch abspaltbare Gruppe, z. B. eine Benzylgruppe oder t-Butylthiogruppe, bevorzugt, wogegen die andere vorzugsweise eine n-Niederalkylthiogruppe, wie Methylthio-, Aethylthio-, Propylthio- oder Butylthiogruppe, ist.

Eine bevorzugte Variante des erfindungsgemässen Verfahrens besteht darin, dass man im Ausgangsstoff der Formel II alle vorhandenen Schutzgruppen abspaltet, was bei geeigneter Wahl von Schutzgruppen mit Vorteil in einer einzigen Operation verwirklicht werden kann, und nachher die als Zwischenprodukt vorliegende Verbindung der Formel II, worin je A, A', B, B', C und C' Wasserstoff und D Hydroxyl oder die Aminogruppe $NH_2$ bedeuten, in an sich bekannter Weise mit einem Oxidationsmittel, wie insbesondere elementarem Jod oder Sauerstoff, behandelt.

Eine besonders bevorzugte Variante des erfindungsgemässen Verfahrens besteht darin, dass man einen Ausgangsstoff der Formel II, worin X, trp, Y, A, A', B, B', C, C' und D die oben definierten Bedeutungen haben, wobei mindestens eines der Symbole A, A', B, B' und D eine Schutzgruppe bedeutet, gegebenenfalls unter gleichzeitiger Abspaltung vorhandener Mercapto-Schutzgruppen zu einem cyclischen disulfidischen Zwischenprodukt der Formel

Bmp-Lys(A)-X-Phe-Phe-trp-Lys(A')-Thr(B)-Phe-Thr(B')-Y-Cys-D (IIA)

(Bmp and Cys joined by a bracket line)

worin X, trp, Y, sowie A, A', B, B' und D die obengenannten Bedeutungen haben, wobei mindestens eines der Symbole A, A', B, B' und D eine Schutzgruppe bedeutet, oxidiert und in diesem die vorhandene(n) Schutzgruppe(n) abspaltet.

Als ε-Amino-Schutzgruppen können alle in der Peptid-Chemie üblichen Amino-Schutzgruppen verwendet werden, wie sie in den entsprechenden Nachschlagwerken, z. B. in Houben-Weyl : Methoden der organischen Chemie ; 4. Auflage, Band 15/I, E. Wünsch (Herausgeber) : Synthese von Peptiden. (Georg Thieme Verlag, Stuttgart ; 1974) zusammenfassend beschrieben werden.

So kann man z. B. reduktiv oder basisch abspaltbare Amino-Schutzgruppen verwenden, z. B. insbesondere die Benzyloxycarbonyl-Gruppe und Benzyloxycarbonyl-Gruppen, die im aromatischen Teil durch Halogenatome, Nitrogruppen, Niederalkoxygruppen und/oder Niederalkylreste substituiert sind, wie die p-Chlor- und p-Brombenzyloxycarbonyl-, p-Nitrobenzyloxycarbonyl-, p-Methoxybenzyloxycarbonyl-, p-Tolyloxycarbonyl-Gruppe oder aber die Isonicotinyloxycarbonylgruppe, weiter auch Acylgruppen, wie p-Toluolsulfonyl, Benzolsulfenyl, o-Nitrobenzolsulfenyl bzw. auch Formyl, Trifluoracetyl oder Phthaloyl.

Eine vorteilhafte ε-Amino-Schutzgruppe ist eine Aethoxycarbonylgruppe, die in β-Stellung eine mit drei Kohlenwasserstoffresten substituierte Silylgruppe, wie Triphenylsilyl-, Dimethyl-butyl-silyl oder vor allem Trimethylsilylgruppe, trägt. Eine solche β-(Trihydrocarbylsilyl)-äthoxycarbonylgruppe, wie eine β-(Triniederalkylsilyl)-äthoxycarbonyl-, z. B. insbesondere die β-(Trimethylsilyl)-äthoxycarbonylgruppe, bildet mit der zu schützenden ε-Aminogruppe eine entsprechende β-Trihydrocarbylsilyl-äthoxycarbonyl-aminogruppe (z. B. die β-Trimethylsilyläthoxycarbonylaminogruppe), welche unter den Bedingungen der sauren Hydrolyse und der Hydrogenolyse beständig ist, aber unter ganz spezifischen, sehr milden Bedingungen sich durch Einwirkung von Fluoridionen abspalten lässt. In dieser Beziehung verhält sie sich analog wie die weiter unten als Carboxyl-Schutzgruppe beschriebene β-Silyläthylestergruppe. (Diese Aehnlichkeit muss bei der Synthese besonders berücksichtigt werden : bis auf Einzelfälle schliesst die Anwendung einer dieser Schutzgruppen die gleichzeitige Anwendung der anderen Schutzgruppe aus.) Weitere Einzelheiten sind weiter unten beim Schutz der Carboxylgruppe durch β-Silyläthylester angegeben.

Ganz besonders bevorzugt sind acidolytisch abspaltbare Gruppen, wie in erster Linie die tert-Butoxycarbonyl-Gruppe und analoge Gruppen, z. B. die tert-Amyloxycarbonyl-, Isopropyloxycarbonyl-, Diisopropylmethoxycarbonyl-, Allyloxycarbonyl-, Cyclopentyloxycarbonyl-, Cyclohexyloxycarbonyl-, d-Isobornyloxycarbonyl- und Adamantyloxycarbonylgruppen, sowie auch Gruppen des Aralkyl-Typs, wie Benzhydryl und Triphenylmethyl (Trityl), oder bestimmte Aralkoxycarbonyl-Gruppen des 2-(p-Biphenylyl)-2-propyloxycarbonyl-Typs, die in der Schweizer Patentschrift 509 266 beschrieben sind.

Als Hydroxyl-Schutzgruppe können alle zu diesem Zwecke in der Peptid-Chemie gebräuchlichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert-Butyl. Ferner kann man aber auch reduktiv oder basisch abspaltbare Hydroxyl-Schutzgruppen verwenden, z. B. Benzylgruppen, die im aromatischen Teil durch Halogen, Nitro und/oder Niederalkoxy substituiert sein können, bzw. Niederalkanoylreste, wie Acetyl, oder Aroylreste, wie Benzoyl. Es ist auch möglich, unter Einhaltung gewisser einschränkender Massnahmen ohne Schutz der Hydroxylgruppen zu verfahren.

Als Carboxyl-Schutzgruppen können die üblichen zu diesem Zwecke gebräuchlichen Gruppen verwendet werden, vgl. das oben zitierte Werk (Houben-Weyl). So werden Carboxylgruppen beispielsweise durch Hydrazidbildung oder durch Veresterung geschützt. Zur Veresterung geeignet sind z. B. niedere, gegebenenfalls substituierte Alkanole wie Methanol, Aethanol, Cyanmethylalkohol, 2,2,2-Trichloräthanol, Benzoylmethylalkohol oder insbesondere tert-Butylalkohol, aber auch ein gegebenenfalls substituierter Benzylalkohol. Eine besonders vorteilhafte Kategorie der substituierten Alkanole sind Aethylalkohole, die in β-Stellung eine trisubstituierte Silylgruppe, wie Triphenylsilyl-, Dimethyl-butyl-silyl- oder vor allem Trimethylsilylgruppe, tragen. Wie z. B. im belgischen Patent Nr. 851 576 beschrieben ist, eignen sich diese Alkohole zum Schutze der Carboxylgruppen deshalb besonders gut, weil die entsprechenden β-Silyläthylester, z. B. β-(Trimethylsilyl)-äthylester, zwar die Stabilität üblicher Alkylester besitzen, sich jedoch unter milden Bedingungen durch Einwirkung von Fluoridionen unter Erhaltung aller anderen Schutzgruppen selektiv abspalten lassen.

Als Mercapto-Schutzgruppen kann man alle zu diesem Zwecke in der Peptid-Chemie gebräuchlichen Gruppen verwenden, wobei die Mercaptogruppen insbesondere durch geeignete Acylierung oder Alkylierung geschützt werden. Zur Acylierung geeignet ist z. B. der Acetyl- oder Benzoylrest, ein Niederalkyl-, z. B. Aethyl, -carbamoyl, oder eine gegebenenfalls, wie oben angegeben, substituierte Benzyloxycarbonyl-Gruppe (Carbobenzoxy-Gruppe). Zur Alkylierung geeignet sind z. B. der tert -Butyl-, Isobutyloxymethyl-, Benzylthiomethyl- oder Tetrahydropyranylrest oder gegebenenfalls durch Halogen, Niederalkoxy oder Nitro substituierte Arylmethylreste wie Benzyl, p-Methoxybenzyl, Diphenylmethyl, Dimethoxybenzhydryl oder ganz besonders Trityl, sowie auch Phenylcyclohexyl (PCH), p-Methoxyphenylcyclohexyl (MPCH), Thienyl(2)-cyclohexyl u. a., vgl. Ber. *101*, 681, (1968). Sehr vorteilhaft ist auch ein Acylaminomethylrest der allgemeinen Formel $R-CO-NH-CH_2-$, worin R-CO- den Rest einer Carbonsäure RCOOH bedeutet, vgl. Tetrahedron Letters *1968* (26), 3 057 und die Deutsche Offenlegungsschrift 2 060 969. Der Acylrest R-CO- kann sich von einer aliphatischen, cycloaliphatischen, aromatischen, araliphatischen oder heterocyclischen Carbonsäure oder von einem Kohlensäuremonoderivat, wie einem Kohlensäuremonoester oder von einer Carbaminsäure, ableiten. Durch das Symbol R ist in erster Linie ein gegebenenfalls substituierter Niederalkylrest repräsentiert, z. B. Methyl-, Aethyl-, Propyl-, Isopropyl-, n-Butyl oder tert-Butylrest, der als Substituenten z. B. Chlor, Trifluormethyl oder die Nitrogruppe enthalten kann. Weiter bedeutet R beispielsweise einen gegebenenfalls substituierten Cycloalkylrest mit 3-8, vorzugsweise 5-6, Ringatomen, wie den Cyclopentyl- oder Cyclohexylrest oder einen gegebenenfalls substituierten aromatischen oder araliphatischen, vorzugsweise monocyclischen Rest, vor allem einen gegebenenfalls substituierten Phenyl- oder Benzylrest, z. B. unsubstituiertes oder im Phenylrest durch Niederalkyl, Niederalkoxy, Halogen oder Nitro substituiertes Phenyl oder Benzyl, oder einen monocyclischen Heterocyclylrest, z. B. Thienyl oder Furyl. Besonders bevorzugt unter den Acylaminomethylgruppen ist die Acetylaminomethylgruppe (Acetamidomethylgruppe). Auf die spezielle Anwendung von Alkylthiogruppen zum Schutze von Mercaptogruppen und Ausbildung der S-S-Brücke wurde oben bereits hingewiesen.

Vorzugsweise werden die Schutzgruppen A, A', B, B' und D so gewählt, dass sie unter ähnlichen Bedingungen abspaltbar sind ; besonders bevorzugt sind dabei die bereits hervorgehobenen acidolytisch abspaltbaren Gruppen. Die Abspaltung aller Schutzgruppen erfolgt dann vorteilhaft in einer einzigen Operation ; es können jedoch auch Gruppen verschiedener Art verwendet werden und jede einzeln abgespalten werden.

Vorzugsweise schützt man vorhandene Carboxylgruppen als tert-Butylester, ε-Aminogruppen durch die tert-Butyloxycarbonylgruppe, die Hydroxylgruppen der Threoninreste, sofern sie überhaupt geschützt werden, als tert-Butyläther, und die Mercaptogruppen durch Trityl-, Acetamidomethyl-, p-Methoxybenzyl- oder Tetrahydropyranylgruppen (Thp). Ausser Acetamidomethyl können alle diese funktionellen Gruppen in *einer* Stufe durch Einwirkung von Säuren (Acidolyse) abgespalten werden. Mercapto-Schutzgruppen vom Typ Trityl, Acetamidomethyl und Tetrahydropyranyl können auch, wenn erwünscht, selektiv unter Beibehaltung der Schutzgruppen vom tert-Butyl-Typ mit Schwermetallsalzen, z. B. Mercuriacetat, und Schwefelwasserstoff abgespalten werden. Man erhält so das geschützte Peptid mit freien Mercaptogruppen. Dieses kann zum geschützten Disulfid der oben charakterisierten Formel IIA, in einer an sich bekannten Weise, z. B. mit Jod, mit Dijodäthan in organischen Lösungsmitteln oder mit

Sauerstoff, insbesondere Luftsauerstoff, wie mit Luftsauerstoff in flüssigem Ammoniak, oxidiert werden. Besonders vorteilhaft ist es, die Mercaptogruppen durch Trityl-, Tetrahydropyranyl- oder Acylaminomethylgruppen zu schützen und diese aus dem geschützten Peptid unter gleichzeitiger Bildung der Disulfidbrücke mit Jod, z. B. in Methanol oder Essigsäure, zu entfernen.

Die Abspaltung der Schutzgruppen erfolgt in an sich bekannter Weise ; die saure Hydrolyse (Acidolyse) wird z. B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z. B. durch Hydrierung unter Palladium-Katalyse, entfernt. Die Isonicotinyloxycarbonylgruppe wird vorzugsweise durch Zink-Reduktion abgespalten.

Die erfindungsgemässen Endstoffe werden, je nach der Art der Isolierung, als Basen oder als Säureadditionssalze erhalten ; diese können nachträglich in an sich bekannter Weise ineinander umgewandelt werden.

Auch die Bildung der oben erwähnten Komplexe erfolgt nach bekannten Methoden : Komplexe mit schwerlöslichen Metall-, z. B. Aluminium oder Zinkverbindungen werden vorzugsweise in analoger Weise, wie für ACTH bekannt, z. B. durch Umsetzung mit einem löslichen Salz des betreffenden Metalls, z. B. Zinckchlorid oder Zinksulfat, und Ausfällung mit einem Alkalimetallphosphat und/oder -hydroxid hergestellt. Komplexe mit organischen Verbindungen wie Polyoxygelatine, Carboxymethylcellulose, Polyvinylpyrrolidon, Polyphloretinphosphat, Polyglutaminsäure etc. werden durch Mischen dieser Substanzen mit dem Peptid in wässeriger Lösung erhalten. In gleicher Weise können auch unlösliche Verbindungen mit Alkalimetallpolyphosphaten hergestellt werden.

Die Ausgangsstoffe der oben charakterisierten Formel II und IIA, und, wenn nicht anders angegeben, auch die zu ihrer Synthese dienenden Zwischenprodukte sind neu und zum Teil auch zur Synthese anderer Somatostatin-Analogen, z. B. solcher mit analogen Aminosäure-Teilsequenzen, mit Vorteil anwendbar. Sie gehören, sowie auch ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CO-NH-Bindungen in beliebiger zeitlicher Reihenfolge miteinander kondensiert, wobei nicht an der Reaktion teilnehmende funktionelle Gruppen intermediär geschützt werden können.

Bei der Herstellung dieser Ausgangsstoffe, wie auch aller benötigten Zwischenprodukte, kommen als Schutzgruppen für die endständigen α-Amino- und Carboxylgruppen besonders die bei der Synthese langkettiger Peptide üblichen Schutzgruppen in Betracht, die z. B. durch Solvolyse oder Reduktion leicht und selektiv abgespalten werden können.

Als α-Amino-Schutzgruppen sind beispielsweise zu nennen : gegebenenfalls, z. B. durch Halogen, Nitro, Niederalkyl oder Niederalkoxy, substituierte Di- oder Triarylniederalkylgruppen, wie Diphenylmethyl oder Triphenylmethylgruppen, z. B. Benzhydryl, Trityl, Di-(p-methoxy)-benzhydryl, oder vor allem von der Kohlensäure sich ableitende hydrogenolytisch abspaltbare Gruppen, wie gegebenenfalls im aromatischen Rest durch Halogenatome, Nitrogruppen, Niederalkyl- oder Niederalkoxygruppen substituierte Benzyloxycarbonylgruppen, z. B. Benzyloxycarbonyl (d. h. Carbobenzoxy), p-Brom- oder p-Chlorbenzyloxycarbonyl, p-Nitrobenzyloxycarbonyl, p-Methoxybenzyloxycarbonyl ; ferner auch 2-(p-Biphenylyl)-2-propyloxycarbonyl und ähnliche im Schweizer Patent 509 266 beschriebene Aryloxycarbonylgruppen. Dabei ist zu beachten, dass die α-Aminoschutzgruppe selektiv unter Erhaltung der gegebenenfalls vorhandenen ε-Aminoschutzgruppe des Lysinrests abspaltbar sein muss. Es ist übrigens auch von Vorteil, wenn bei ihrer Abspaltung auch eine gegebenenfalls vorhandene Carboxyl- und Hydroxylschutzgruppe unversehrt bleibt.

Die Carboxyl-Schutzgruppen für diesen Zweck sind dieselben, wie sie oben bei der entsprechenden Bedeutung des Symbols D besprochen wurden.

Diese Schutzgruppen können in bekannter Weise abgespalten werden. So kann man die Benzyloxycarbonylgruppe durch Hydrogenolyse, die N-Tritylgruppe mit Mineralsäuren, wie Halogenwasserstoffsäuren, z. B. Fluorwasserstoff oder vorzugsweise Chlorwasserstoff, oder einer organischen Säure, wie Ameisensäure, Essigsäure, Chloressigsäure oder Trifluoressigsäure, in wässerigem oder absolutem Trifluoräthanol als Lösungsmittel (vgl. Deutsche Offenlegungsschrift DT 2 346 147) oder mit wässeriger Essigsäure ; die tert-Butyloxycarbonylgruppe mit Trifluoressigsäure oder Salzsäure, die 2-(p-Biphenylyl)-isopropyloxycarbonylgruppe mit wässeriger Essigsäure oder z. B. mit einem Gemisch von Eisessig, Ameisensäure (82,8 %ig) und Wasser (7 : 1 : 2) oder nach dem Verfahren der DT 2 346 147 abspalten.

Die β-Silyläthylestergruppen werden vorzugsweise mit Fluoridionen-abgebenden Reagentien, z. B. Fluoriden quaternärer-organischer Basen, wie Tetraäthylammoniumfluorid, abgespalten. Sie können aber auch, gleich wie die üblichen Alkylester, durch alkalische Hydrolyse, z. B. mittels Alkalimetallhydroxiden, -carbonaten oder -bicarbonaten, abgespalten werden oder durch Hydrazinolyse, z. B. mittels Hydrazinhydrat, in die entsprechenden Carbazoylgruppen umgewandelt werden. Zur Abspaltung von tert-Butylestern verwendet man vorzugsweise Acidolyse, für Benzylester dann Hydrogenolyse.

Die zur Herstellung der Ausgangsstoffe der Formel II durchzuführende Kondensation der Aminosäure- und/oder Peptideinheiten erfolgt in an sich bekannter Weise, indem man vorzugsweise eine Aminosäure

oder ein Peptid mit geschützter α-Aminogruppe und gegebenenfalls aktivierter terminaler Carboxylgruppe (= aktive Komponente) an eine Aminosäure oder ein Peptid mit freier α-Aminogruppe und freier oder geschützter z. B. veresterter terminaler Carboxylgruppe (= passive Komponente), anknüpft, im gebildeten Produkt die terminale Aminogruppe freisetzt und dieses Peptid, enthaltend eine freie α-Aminogruppe und eine gegebenenfalls geschützte terminale Carboxylgruppe, wieder mit einer weiteren aktiven Komponente, d. h. einer Aminosäure oder einem Peptid mit aktivierter terminaler Carboxylgruppe und freier α-Aminogruppe, umsetzt, usw. Die Carboxylgruppe kann beispielsweise durch Ueberführung in ein Säureazid, -anhydrid, -imidazolid, -isoxazolid oder einen aktivierten Ester, wie einen der weiter unten genannten, oder durch Reaktion mit einem Carbodiimid, wie N,N'-Dicyclohexylcarbodiimid, gegebenenfalls unter Zusatz von N-Hydroxysuccinimid, einem unsubstituierten oder z. B. durch Halogen, Methyl oder Methoxy substituierten 1-Hydroxybenzotriazol oder 4-Hydroxybenzo-1,2,3-triazin-3-oxid (u. a., vgl. DT 1 917 690, DT 1 937 656, DT 2 202 613), oder insbesondere vom N-Hydroxy-5-norbornen-2,3-dicarboximid, oder mit N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode, sowie die Merrifield-Methode und die Methode der N-Carboxyanhydride oder N-Thiocarboxyanhydride.

Zur Bildung von aktivierten Estern, wie oben erwähnt sind, eignen sich z. B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Penta(fluor oder chlor)phenol, o- und p-Nitrophenol, 2,4-Dinitrophenol, p-Cyanophenol, weiter z. B. N-Hydroxysuccinimid, N-Hydroxyphthalimid und N-Hydroxypiperidin.

Bei einer besonders bevorzugten Herstellung der Peptide der Formel II verwendet man als Kupplungsmethode die Carbodiimid-Methode mit N,N'-Dicyclohexylcarbodiimid in Anwesenheit von 1-Hydroxybenzotriazol. Die terminale Carboxylgruppe wird dabei in Form des β-(Trimethyl-silyl)-äthylesters geschützt, die α-Aminogruppe der aktiven Komponente wird durch die Benzyloxycarbonylgruppe geschützt, die nach jedem Kupplungsschritt durch Hydrogenolyse abgespalten wird. Zum Schutz der ε-Aminogruppe der Lysinreste wird die Acylierung mit der tert-Butoxycarbonylgruppe und für die Hydroxylgruppe der Threoninreste die Verätherung mit der tert-Butylgruppe verwendet. Diese beiden Schutzgruppen können, wenn erwünscht, zuletzt in einem Schritt durch saure Hydrolyse, z. B. mittels Trifluoressigsäure, Salzsäure oder Fluorwasserstoff, abgespalten werden.

Beide schwefelhaltigen Säurereste ($Bmp^3$ und $Cys^{14}$) führt man vorzugsweise erst in den letzten Stadien der Synthese ein, da die Anwesenheit von Schwefel bekanntlich die Aktivität von Hydrierungskatalysatoren beeinträchtigen kann und somit die Anwendung der sonst sehr vorteilhaften hydrogenolytisch abspaltbaren Gruppen in Frage stellt. Die Mercaptogruppen in den besagten Säuren werden mit Vorteil durch die Tritylgruppen geschützt, welche für die Durchführung bevorzugter Verfahrensvarianten besonders geeignet sind.

Je nach Arbeitsweise erhält man die Verbindungen in Form von Basen oder ihren Salzen. Aus den Salzen können in an sich bekannter Weise die Basen gewonnen werden. Von letzteren wiederum lassen sich durch Umsetzen mit Säuren, z. B. mit solchen, die die oben genannten Salze bilden, therapeutisch anwendbare Säureadditionssalze gewinnen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen der Verfahren, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze davon enthalten. Diese pharmazeutischen Präparate können insbesondere in den oben angegebenen Indikationen Verwendung finden, wenn sie intraperitoneal, wie intravenös, intramuskulär oder subcutan, oder auch intranasal verabreicht werden. Die erforderliche Dosis hängt von der speziellen zu behandelnden Erkrankung, ihrer Schwere und von der Dauer der Therapie ab. Anzahl und Menge der Einzeldosen, sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,001 5 bis etwa 0,15 mg/kg Körpergewicht und die Verabreichung erfolgt durch intravenöse Infusion oder subkutane Injektion. Dementsprechend enthalten pharmazeutische Präparate zur parenteralen Verabreichung in Einzeldosis-Form in Abhängigkeit von der Applikationsart pro Dosis etwa 0,08 bis etwa 15 mg einer der erfindungsgemässen Verbindungen. Neben dem Wirkstoff enthalten sie üblicherweise noch einen Puffer, z. B. einen Phosphatpuffer, der das pH zwischen etwa 3,5 und 7 halten soll, ferner Natriumchlorid, Mannit

oder Sorbit zur Einstellung der Isotonie. Sie können in gefriergetrockneter oder gelöster Form vorliegen, wobei Lösungen mit Vorteil ein antibakteriell wirkendes Konservierungsmittel, z. B. 0,2-0,3 % 4-Hydroxybenzoesäure-methylester oder -äthylester enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z. B. gemäss der oben erwähnten Massnahmen zubereitet werden, gebildet werden. Als Zusatz eignet sich z. B. 0,1-1,0 Gewichts-% eines Zink(II)-Salzes (z. B. Sulfats) in Verbindung mit 0,5-5,0 Gewichts-% Protamin (z. B. als Sulfat), bezogen auf das Gesamtvolumen der Injektionslösung ; diese Zubereitung liegt als Lösung von pH 3,5 bis etwa 6,5 oder als Suspension von etwa pH 7,5 bis 8,0 vor.

Ein Präparat für die intranasale Verabreichung kann als eine wässrige Lösung oder Gelee, eine ölige Lösung oder Suspension, oder aber eine fetthaltige Salbe vorliegen. Ein Präparat in Form einer wässrigen Lösung erhält man z. B. dadurch, dass man den Wirkstoff der Formel I, oder ein therapeutisch anwendbares Säureadditionssalz davon, in einer wässrigen Pufferlösung von pH bis 7,2 löst und einen Isotonie-erzeugenden Stoff zusetzt. Der wässrigen Lösung wird zweckmässig ein polymeres Haftmittel, z. B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z. B. durch Suspendieren eines Peptids der Formel I oder eines therapeutisch anwendbaren Säureadditionssalzes davon in einem Oel, gegebenenfalls unter Zusatz von Quellmitteln, wie Aluminiumstearat, und/oder grenzflächenaktiven Mitteln (Tensiden), deren HLB-Wert (« hydrophilic-lipophilic-balance » unter 10 liegt, wie Fettsäuremonoester mehrwertiger Alkohole, z. B. Glycerinmonostearat, Sorbitanmonolaurat, Sorbitanmonostearat oder Sorbitanmonoleat. — Eine fetthaltige Salbe erhält man z. B. durch Suspendieren des erfindungsgemässen Wirkstoffes in einer streichbaren Fettgrundlage, gegebenenfalls unter Zusatz eines Tensids vom HLB-Wert unter 10. Eine Emulsionssalbe erhält man durch Verreiben einer wässrigen Lösung des peptidischen Wirkstoffes in einer weichen, streichbaren Fettunterlage unter Zusatz eines Tensids, dessen HLB-Wert unter 10 liegt. Alle diese intranasale Applikationsformen können auch Konservierungsmittel enthalten. — Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puderverteilenden Eigenschaften enthalten ausser dem Wirkstoff üblicherweise Hilfsmittel : Insufflationskapseln enthalten z. B. feste Trägerstoffe, wie Lactose ; Aerosol- bzw. Sprayzubereitungen enthalten z. B. ein flüssiges Treibgas mit einem Siedepunkt unter der Raumtemperatur, sowie, wenn erwünscht, weitere Trägerstoffe, wie flüssige oder feste nicht-ionische oder anionische Tenside und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbidungen der Formel I und therapeutisch anwendbaren Säureadditionssalzen davon als pharmakologisch aktive Verbindungen, insbesondere in den für Somatostatin üblichen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen wird die Erfindung illustriert, jedoch durch diese nicht eingeschränkt. Temperaturen werden in Celsiusgraden angegeben ; als Abkürzungen, z. B. zur Bezeichnung von Aminosäuren, Peptiden, Schutzgruppen etc., werden die übliche, z. B. die in « Synthese von Peptiden » (Herausgeber : E. Wünsch), Bd XV der « Methoden der org. Chemie » (Houben-Weyl) (1974 ; G. Thieme, Stuttgart), zusammengestellten Kurzbezeichnungen verwendet. Insbesondere werden folgende Abkürzungen verwendet.

| | |
|---|---|
| Bmp | — β-Mercaptopropionsäure |
| Boc | — tert-Butoxycarbonyl |
| Bpoc | — 2-(p-Biphenylyl)-2-propyloxycarbonyl |
| But | — tert-Butyl (als ätherbildende Gruppe) |
| HONB | — N-Hydroxy-5-norbornen-endo-2,3-dicarboximid |
| OBut | — tert-Butoxy (als esterbildende Gruppe) |
| ONP | — p-Nitrophenoxy (als esterbildende Gruppe) |
| OTmse | — 2-(Trimethylsilyl)-äthoxy (als esterbildende Gruppe) |
| Trt | — Triphenylmethyl (=Trityl) |
| Z | — Benzyloxycarbonyl (Carbobenzoxy) |
| DC | — Dünnschichtchromatographie |

In DC verwendet man, wenn nichts anderes angegeben ist, Kieselgel als Adsorbens und die folgenden Systeme als Laufmittel :

System 45 : sec-Butylalkohol-3 %-wässr. Ammoniak (70 : 30)
101 : Butanol-Pyridin-Essigsäure-Wasser (38 : 24 : 8 : 30)
155 : Pentanol-Pyridin-Wasser-Butanon-Essigsäure (40 : 28 : 15 : 11 : 5)
157 : Chloroform-Methanol-Essigsäure-Wasser (70 : 42 : 0,5 : 10)

Beispiel 1

Bmp-Lys-Asn-Phe-Phe-(D-Trp)-Lys-Thr-Phe-Thr-Pro-Cys-OH

190 mg Bmp-Lys(Boc)-Asn-Phe-Phe-(D-Trp)-Lys(Boc)-Thr(But)-Phe-Thr(But)-Pro-Cys-OBut werden bei 0° in 3,0 ml Trifluoressigsäure-Wasser (9 : 1) aufgenommen und nach 30 Minuten bei 20° mit 30 ml Aether ausgefällt. Das rohe Trifluoracetat wird im Vakuum getrocknet, in 5 ml IN Essigsäure gelöst und durch 15 ml Anionenaustauscher in Acetatform (z. B. AG®1-X8, ein Produkt von Bio-Rad Laboratories, Richmond, Calif., USA) filtriert. Das Eluat wird im Vakuum eingedampft und der Rückstand der Gegenstromverteilung im System n-Butanol-Wasser-Eisessig (4 : 5 : 1) unterworfen. Nach 800 Verteilungsschritten befindet sich das Produkt in den Elementen 170-200 (K = 0,26). Die Titelverbindung ist dünnschichtchromatographisch einheitlich.
DC : System 157 : Rf 0,15
Der Ausgangsstoff wird folgendermassen hergestellt :

Stufe 1.1 Z-Phe-Thr(But)-OTmse

Eine Lösung von 101,82 g Z-Phe-OH und 90,91 g H-Thr(But)-OTmse in 660 ml Methylenchlorid wird mit 81,65 g Dicyclohexylcarbodiimid versetzt. Nach 15 Stunden bei 20° wird der Niederschlag abfiltriert, das Filtrat auf die Hälfte ihres Volumens eingeengt und bis zur Trübung mit Petroläther versetzt. Die Titelverbindung kristallisiert in feinen Nadeln aus. Smp. 92-93°. $[\alpha]_D = -1°$.

Stufe 1.2 H-Phe-Thr(But)-OTmse

Eine Lösung von 10 g Z-Phe-Thr(But)-OTmse (Stufe 1.1) in 100 ml Methanol wird auf 1,0 g Pd-Kohle (10 %-ig) während einer Stunde hydriert. Das Reaktionsgemisch wird vom Katalysator abfiltriert und das Lösungsmittel eingedampft, womit als Rückstand die Titelverbindung erhalten wird.
DC : Tetrachlorkohlenstoff-Aethylacetat (6 : 4) : Rf 0,15.

Stufe 1.3 Z-Thr(But)-Phe-Thr(But)-Otmse

Ein Gemisch von 6,12 g Z-Thr(But)-OH, 7,62 g H-Phe-Thr(But)-OTmse (Stufe 1.2) und 4.48 g Dicyclohexylcarbodiimid in 50 ml Methylenchlorid wird 20 Stunden bei 20° stehen gelassen und filtriert. Das Filtrat wird eingeengt und die verbliebene Lösung tropfenweise mit Petroläther versetzt. Das ausgefallene Produkt wird durch nochmaliges Umfällen aus Aethylacetat-Petroläther rein erhalten.
DC : Tetrachlorkohlenstoff-Aethylacetat (6 : 4) : Rf 0,59.

Stufe 1.4 H-Thr(But)-Phe-Thr(But)-OTmse

Eine Lösung von 1,43 g Z-Thr(But)-Phe-Thr(But)-OTmse (Stufe 1.3) in 14 ml Methanol wird nach Zugabe von 0,15 g Pd-Kohle (10 %-ig) während 2 Stunden hydriert. Der Katalysator wird abfiltriert und aus dem Filtrat das Lösungsmittel abgedampft, womit als Rückstand die Titelverbindung resultiert.
DC : Chloroform-Methanol (95 : 5) : Rf 0,38.

Stufe 1.5 Z-(D-Trp)-Lys(Boc)-Thr(But)-Phe-Thr(But)-OTmse

Zu einer Lösung von 1,15 g H-Thr(But)-Phe-Thr(But)-OTmse (Stufe 1.4) und 1,24 g Z-(D-Trp)-Lys(Boc)-OH in 10 ml Acetonitril gibt man 0,35 g N-Hydroxybenzotriazol und 0,472 g Dicyclohexylcarbodiimid. Nach 20 Stunden bei 20° wird der ausgeschiedene Niederschlag abfiltriert, das Filtrat eingedampft und der Rückstand aus heissem Aethylacetat umgefällt.
DC : Cyclohexan-Aceton (7 : 3) : Rf 0,20.

Stufe 1.6 H-(D-Trp)-Lys(Boc)-Thr(But)-Phe-Thr(But)-OTmse

Eine Lösung von 7,9 g Z-(D-Trp)-Lys(Boc)-Thr(But)-Phe-Thr(But)-OTmse (Stufe 1.5) in 200 ml

Methanol wird nach Zugabe von 1 g Pd-Kohle (10 %-ig) während 2 Stunden hydriert. Die Reaktionslösung wird vom Katalysator abfiltriert und das Lösungsmittel eingedampft, womit als Rückstand die Titelverbindung resultiert.

DC : Chloroform-Methanol (8 : 2) : Rf 0,60.

Stufe 1.6A H-Asn-Phe-Phe-OH

Eine Lösung von 20,5 g Z-Asn-Phe-Phe-OH (siehe Case 4-11 346, Stufe 1.5A) in 270 ml Methanol und 30 ml Wasser wird bei Raumtemperatur auf 2 g Pd-Kohle (10 %-ig) während 4 Stunden hydriert. Nach dem Abfiltrieren des Katalysators wird das Filtrat im Vakuum eingedampft und der Rückstand direkt in der nächsten Stufe eingesetzt.

Stufe 1.6B Z-Lys(Boc)-Asn-Phe-Phe-OH

Eine Suspension von 15,6 g H-Asn-Phe-Phe-OH (Stufe 1.6A) und 27,6 g Z-Lys(Boc)-ONP in 200 ml Dimethylformamid, 25 ml Wasser und 9,2 ml 4N Natronlauge wird während 20 Stunden bei Raumtemperatur gerührt, anschliessend durch Zugabe von 36,8 ml 1N Salzsäure bei 0-5° neutralisiert und mit 300 ml Wasser versetzt, womit das Rohprodukt ausfällt. Dieses wird getrocknet, zweimal mit je 300 ml Aether verrührt und nochmals getrocknet. Die Ausbeute beträgt 88 % eines gemäss Dünnschichtchromatographie einheitlichen Produktes.

DC : Butanol-Essigsäure-Wasser (3 : 1 : 1) : Rf 0,82 ; Methyläthylketon-Pyridin-Wasser (65 : 5 : 20) : Rf 0,53.

Stufe 1.7 Z-Lys(Boc)-Asn-Phe-Phe-(D-Trp)-Lys(Boc)-Thr(But)-Phe-Thr-(But)-OTmse

Eine Lösung von 5,84 g Z-Lys(Boc)-Asn-Phe-Phe-OH (Stufe 1.6B) und 6,64 g H-(D-Trp)-Lys(Boc)-Thr(But)-Phe-Thr(But)-OTmse (Stufe 1.6) in 30 ml Dimethylformamid wird mit 1,123 g N-Hydroxybenzotriazol und 1,72 g Dicyclohexylcarbodiimid versetzt. Nach 20 Stunden bei 20° filtriert man ab und giesst das Filtrat in 150 ml Eiswasser. Der Niederschlag wird abfiltriert und der getrocknete Rückstand zweimal aus Acetonitril-Ethanol (8 : 2) umgefällt, womit die Titelverbindung resultiert.

DC : Chloroform-Methanol-Wasser-Eisessig (88 : 10,5 : 1 : 0,5) : Rf 0,44.

Stufe 1.8 H-Lys(Boc)-Asn-Phe-Phe-(D-Trp)-Lys(Boc)-Thr(But)-Phe-Thr-(But)-OTmse

Eine Lösung von 3,53 g Z-Lys(Boc)-Asn-Phe-Phe-(D-Trp)-Lys(Boc)-Thr(But)-Phe-Thr(But)-OTmse (Stufe 1.7) in 100 ml 90 %igem Trifluorethanol wird nach Zugabe von 0,4 g Pd-Kohle (10 %-ig) während 3 Stunden bei Raumtemperatur hydriert, die Reaktionslösung vom Katalysator abfiltriert und das Lösungsmittel abgedampft. Die als Rückstand erhaltene Titelverbindung zeigt im Dünnschichtchromatogramm auf Silikagel im System Chloroform-Methanol-Wasser-Eisessig (88 : 10,5 : 1 : 0,5) einen Rf-Wert von 0,24.

Stufe 1.9 Bmp(Trt)-Lys(Boc)-Asn-Phe-Phe-(D-Trp)-Lys(Boc)-Thr(But)-Phe-Thr(But)-OTmse.

Zu 0,353 g Bmp(Trt)-OH und 1,50 g H-Lys(Boc)-Asn-Phe-Phe-(D-Trt)-Lys(Boc)-Thr(But)-Phe-Thr(But)-OTmse (Stufe 1.8) in 10 ml Dimethylformamid gibt man 0,155 g N-Hydroxybenzotriazol und 0,246 g Dicyclohexylcarbodiimid. Nach 15 Stunden bei 20° filtriert man ab und giesst das Filtrat in 100 ml Eiswasser. Es wird abfiltriert und aus Methanol umgefällt.

DC : Chloroform-Methanol (9 : 1) : Rf 0,66 ;
Chloroform-Methanol-Wasser-Eisessig (88 : 10,5 : 1 : 0,5) : Rf 0,71.

Stufe 1.10 Bmp(Trt)-Lys(Boc)-Ans-Phe-Phe-(D-Trp)-Lys(Boc)-Thr(But)-Phe-Thr(But)-OH

1,35 g Bmp(Trt)-Lys(Boc)-Asn-Phe-Phe-(D-Trp)-Lys(Boc)-Thr(But)-Phe-Thr(But)-OTmse (Stufe 1.9) werden in 23 ml 0,15N-Lösung von Tetra-äthylammoniumfluorid in Dimethylformamid aufgenommen. Nach 35 Minuten bei 30° wird die Lösung unter Eiskühlung zu einem Gemisch von 90 ml Wasser und 2,16 ml IN Salzsäure gegossen, abfiltriert, reichlich mit Wasser gewaschen und getrocknet. Die Titelverbindung wird als weisses Pulver in dünnschichtchromatographisch reiner Form erhalten.

DC : Chloroform-Isopropanol-Eisessig (70 : 8 : 2) : Rf 0,28 ;
Chloroform-Methanol (9 : 1) : Rf 0,20.

Stufe 1.10A Bpoc-Pro-Cys(Trt)-OBut

Ein Gemisch von 1,77 g Bpoc-Pro-OH und 0,63 ml N-Aethylmorpholin in 35 ml Tetrahydrofuran versetzt man bei −10° mit 0,665 ml Isobutylchlorocarbonat, und nach 15 Minuten bei −10° tropft man eine Lösung von 2,135 g H-Cys(Trt)-OBut in 25 ml Tetrahydrofuran hinzu, lässt 1 Stunde bei −10° und 15 Stunden bei 20° reagieren und dampft ein. Der Rückstand wird in 100 ml Aethylacetat aufgenommen, die

Lösung nacheinander mit 1N Zitronensäure-Lösung, 1N Natriumhydrogencarbonat-Lösung und Wasser extrahiert, über Natriumsulfat getrocknet und eingedampft. Das Produkt wird durch Chromatographie an einer Säule von Silicagel rein erhalten.

DC : Toluol-Aceton (7 : 3) : Rf 0,60 ;
Aethylacetat-Petroläther (1 : 1) : Rf 0,40.

Stufe 1.10B H-Pro-Cys(Trt)-OBut

Zu einer Lösung von 1,13 g Bpoc-Pro-Cys(Trt)-OBut (Stufe 1.10A) in 25 ml Eisessig gibt man tropfenweise 3 ml Wasser. Nach einer Stunde bei 45° wird eingedampft, der Rückstand in 100 ml Aethylacetat aufgenommen und die Lösung nacheinander mit 1N Natriumhydrogencarbonat-Lösung und Wasser gewaschen, über Natriumsulfat getrocknet und eingedampft. Das erhaltene Produkt [DC : Toluol-Aceton (7 : 3) : Rf 0,20] wird direkt in der folgenden Kondensation eingesetzt.

Stufe 1.11 Bmp(Trt)-Lys(Boc)-Asn-Phe-Phe-(D-Trp)-Lys(Boc)-Thr(But)-Phe-Thr(But)-Pro-Cys(Trt)-OBut

Zu einer Lösung von 696 mg Bmp(Trt)-Lys(Boc)-Asn-Phe-Phe-(D-Trp)-Lys-(Boc)-Thr(But)-Phe-Thr(But)-OH (Stufe 1.10), 266 mg H-Pro-Cys(Trt)-OBut (Stufe 1.10B) und 91 mg N-Hydroxybenzotriazol in 3 ml Dimethylformamid gibt man 123 mg Dicyclohexylcarbodiimid. Nach 15 Stunden bei 20° wird abfiltriert, das Filtrat eingedampft und der Rückstand aus Isopropanol umgefällt. Man erhält die Titelverbindung in dünnschichtchromatographisch reiner Form in einer Ausbeute von 85 %.

DC : Chloroform-Methanol (9 : 1) : Rf 0,70.

Stufe 1.12 Bmp-Lys(Boc)-Asn-Phe-Phe-(D-Trp)-Lys(Boc)-Thr(But)-Phe-Thr(But)-Pro-Cys-OBut

660 mg Bmp(Trt)-Lys(Boc)-Asn-Phe-Phe-(D-Trp)-Lys(Boc)-Thr(But)-Phe-Thr(But)-Pro-Cys(Trt)-OBut (Stufe 1.11) werden in 30 ml Dimethylformamid gelöst und zur gerührten Lösung von 711 mg Jod in 400 ml Methanol innert 15 Minuten zugetropft. Nach weiteren 10 Minuten giesst man eine Lösung von 556 mg Ascorbinsäure in 27 ml Wasser zu, engt die farblose Lösung auf ca. 20 ml ein und fällt mit 100 ml Wasser aus. Der Rückstand wird getrocknet und einer Gegenstromverteilung im System Methanol-Puffer (28.6 ml Eisessig, 19.3 g Ammoniumacetat, 1 Liter Wasser)-Chloroform-Tetrachlorkohlenstoff (4 : 1 : 2 : 2) unterworfen. Nach 900 Verteilungsschritten befindet sich die Substanz in den Elementen 160-195 (K~0,22).

DC : Chloroform-Methanol (9 : 1) : Rf 0,40 ;
Chloroform-Isopropanol-Eisessig (70 : 8 : 2) : Rf 0,30.

## Beispiel 2

Bmp-Lys-Asn-Phe-Phe-(D-Trp)-Lys-Thr-Phe-Thr-Pro-Cys-OH

100 mg Bmp(Trt)-Lys(Boc)-Asn-Phe-Phe-(D-Trp)-Lys(Boc)-Thr(But)-Phe-Thr-(But)-Pro-Cys(Trt)-OBut (Stufe 1.11) werden bei 0° in 1,0 ml konzentrierter Salzsäure gelöst und nach 2 Minuten bei 0° mit 50 ml Wasser versetzt. Nach dreimaligem Extrahieren mit je 50 ml Aether wird die wässerige Phase durch Zugabe von 12,5 ml 1N Natronlauge auf pH 7,4 gestellt und während 24 Stunden Luft durchgeleitet. Das Reaktionsgemisch wird im Vakuum eingedampft, der Rückstand in 3 ml Essigsäure gelöst und durch 70 ml Anionenaustauscher in Acetatform (z. B. AG® 1-X8, ein Produkt von Bio-Rad Laboratories, Richmond, Calif., USA.) filtriert. Das Eluat wird im Vakuum eingedampft und der Rückstand der Gegenstromverteilung im System n-Butanol-Wasser-Eisessig (4 : 5 : 1) unterworfen. Nach 800 Verteilungsschritten befindet sich das Produkt in den Elementen 165-195 (K~0,23). Gemäss Dünnschichtchromatographie ist das Produkt einheitlich.

DC : System 157 : Rf 0,15.

## Beispiel 3

Bmp-Lys-Asn-Phe-Phe-(D-Trp)-Lys-Thr-Phe-Thr-Tpo-Cys-OH

(Tpo = 4-Thiaprolin)

173 mg Bmp-Lys(Boc)-Asn-Phe-Phe-(D-Trp)-Lys(Boc)-Thr(But)-Phe-Thr(But)-Tpo-Cys-OBut werden bei 0° in 9,0 ml Trifluoressigsäure-Wasser (9 : 1) aufgenommen und nach 30 Minuten bei 20° mit 50 ml

Aether ausgefällt. Das rohe Trifluoracetat wird im Vakuum getrocknet und der Gegenstromverteilung im System n-Butanol-Wasser-Eisessig (4 : 5 : 1) unterworfen. Nach 386 Verteilungsschritten befindet sich das Produkt in den Elementen 225-280 (K = 1,58). Die Titelverbindung ist dünnschicht-chromatographisch einheitlich.

DC : System 45 : Rf 0,15
101 : Rf 0,47
155 : Rf 0,60

Der Ausgangsstoff wird folgendermasser hergestellt :

Stufe 3.1 Boc-Tpo-OH (als Cyclohexylammoniumsalz)

Eine Suspension von 2,66 g 4-Thiaprolin und 2,12 g $Na_2CO_3$ in einem Gemisch von 20 ml $H_2O$ und 40 ml Dioxan wird mit 4,80 g Di-tert-butyldicarbonat versetzt. Nach 30 Minuten bei 20° wird auf 0°-5 °C abgekühlt und mit 17,5 ml 2-N HCl auf pH 2,0 gestellt, die Lösung mit Aethylacetat extrahiert, das Aethylacetat-Extrakt getrocknet und auf Volumen von 20 ml eingedampft. Anschliessend wird bei 0°-5 °C 2,30 ml Cyclohexylamin dazupipettiert, kristalliner Niederschlag abfiltriert und getrocknet.

F = 202°-203 °C

$[\alpha]_D$ : $-$ 105 ± 1° (c = 0,59 % in $CHCl_3$)

Stufe 3.2 Boc-Tpo-OTmse

Eine Lösung von 2,33 g Boc-Tpo-OH (als Cyclohexylammoniumsalz, Stufe 3.1), 1,61 ml Pyridin und 1,59 ml 2-Trimethylsilyl-äthanol in 30 ml Acetonitril wird bei $-5°$ innerhalb 15 Minuten portionenweise mit 2,27 g Dicyclohexylcarbodiimid versetzt. Nach 15 Stunden bei 5 °C wird der Niederschlag abfiltriert, das Filtrat eingedampft, in 200 ml Aethylacetat aufgenommen, nacheinander mit 1-N HCl, Wasser, 1-N Natriumhydrogencarbonat-Lösung und Wasser extrahiert, über Natriumsulfat getrocknet und eingedampft.

DC : Chloroform-Methanol (8 : 2) : Rf 0,7

Stufe 3.3 H-Tpo-OTmse (Hydrochlorid)

Eine Lösung von 1,64 g Boc-Tpo-OTmse (Stufe 3.2) in 4 ml Aethylacetat wird bei 0 °C mit 23,4 ml 2,1-N HCl in Aethylacetat versetzt. Nach 1 1/4 Stunden bei 20° wird auf 0 °C abgekühlt und mit 200 ml Petroläther gefällt.

DC : Toluol-Aceton (7 : 3) : Rf 0,58

Stufe 3.3A [Bpoc-Thr(But)-]$_2$O

Eine Lösung von 2,48 g Bpoc-Thr(But)-OH in 25 ml Chloroform wird mit 0,62 g Dicyclohexylcarbodiimid versetzt. Nach 15 Stunden bei 20 °C, wird der Niederschlag abfiltriert und das Filtrat eingedampft.

DC : Chloroform-Methanol (8 : 2) : Rf 0,85

Stufe 3.4 Bpoc-Thr(But)-Tpo-OTmse

Eine Lösung von 0,74 g H-Tpo-OTmse (als Hydrochlorid, Stufe 3.3), 347 µl N-Aethylmorpholin und 2, 66 g [Bpoc-Thr(But)]$_2$-O (Stufe 3.3A) in 17 ml Dimethylformamid wird während 15 Stunden bei 20° belassen. Die Lösung wird eingedampft, der Rückstand in Aethylacetat gelöst und mit Wasser extrahiert. Nach dem Trocknen über Natriumsulfat wird das Produkt eingedampft und durch Chromatographie an einer Silicagel-Säule rein erhalten.

DC : Toluol-Aceton (7 : 3) : Rf 0,73

DC : Chloroform-Methanol (8 : 2) : Rf 0,81

Stufe 3.5 Bpoc-Thr(But)-Tpo-OH

Eine Lösung von 1,0 g Bpoc-Thr(But)-Tpo-OTmse (Stufe 3.4) in 2 ml Dimethylformamid wird mit einer 0,7-mol. Tetraäthylammoniumfluoridlösung in Dimethylsulfoxid versetzt. Nach 1 1/2 Stunden bei 20 °C wird auf 0° gekühlt und mit 100 ml 0,01-N HCl ausgefällt.

DC : Chloroform-Methanol (8 : 2) : Rf 0,32

Stufe 3.6 Bpoc-Thr(But)-Tpo-Cys(Trt)-OBut

Eine Lösung von 382 mg Bpoc-Thr(But)-Tpo-OH (Stufe 3.5), 331 mg H-Cys(Trt)-OBut und 141 mg N-Hydroxybenzotriazol in 7 ml Dimethylformamid wird mit 117 mg Dicyclohexylcarbodiimid versetzt. Nach 15 Stunden bei 20° wird der Niederschlag abfiltriert und das Filtrat eingedampft. Der Rückstand wird in Aethylacetat gelöst, mit Wasser gewaschen, über Natriumsulfat getrocknet und eingedampft. Das

Produkt wird durch Säulenchromatographie an Silicagel gereinigt.
DC : Tetrachlormethan-Aethylacetat (6 : 4) : Rf 0,57

Stufe 3.7 H-Thr(But)-Tpo-Cys(Trt)-OBut · Hydrochlorid

Eine Lösung von 307 mg Bpoc-Thr(But)-Tpo-Cys(Trt)-OBut in 20 ml 90 %-igem 2,2,2-Trifluoräthanol wird bei 20° während 35 Minuten durch langsames Zugeben von 276 µl 1-N HCl bei pH 1,0 gehalten. Das Gemisch wird mit Pyridin auf pH 4.3 gestellt und aus tert-Butanol lyophilisiert.
DC : Chloroform-Methanol (8 : 2) : Rf = 0,61

Stufe 3.7A Z-Lys(Boc)-Asn-Phe-Phe-(D-Trp)-Lys(Boc)-Thr(But)-Phe-OTmse

Eine Lösung von 3,25 g Z-Lys(Boc)-Asn-Phe-Phe-OH (Stufe 1.6B) und 3,44 g H-(D-Trp)-Lys(Boc)-Thr(But)-Phe-OTmse in 70 ml Dimethylformamid wird mit 0,81 g HONB und 1,02 g Dicyclohexylcarbodiimid versetzt. Nach 15 Stunden bei 20° wird der Niederschlag abfiltriert und das Filtrat eingedampft. Der Rückstand wird mit einem Gemisch von 50 ml Methanol und 150 ml Aethylacetat verrieben, abgenutscht und getrocknet.
DC : Chloroform Methanol (8 : 2) : Rf 0,87

Stufe 3.7B H-Lys(Boc)-Asn-Phe-Phe-(D-Trp)-Lys(Boc)-Thr(But)-Phe-OTmse

Eine Lösung von 5,0 g Z-Lys(Boc)-Asn-Phe-Phe-(D-Trp)-Lys(Boc)-Thr(But)-Phe-OTmse (Stufe 3.7A) in 80 ml 90 %-igem Trifluorethanol wird nach Zugabe von 1,0 g Pd-Kohle (10 %-ig) während 17 Stunden bei Raumtemperatur hydriert, die Reaktionslösung vom Katalysator abfiltriert und das Lösungsmittel abgedampft. Als Rückstand erhält man die Titelverbindung.
DC : Chloroform-Methanol (8 : 2) : Rf 0,54

Stufe 3.7C Bmp(Trt)-Lys(Boc)-Asn-Phe-Phe-(D-Trp)-Lys(Boc)-Thr(But)-Phe-OTmse

Zu 1,19 g Bmp(Trt)-OH und 4,58 g H-Lys(Boc)-Asn-Phe-Phe-(D-Trp)-Lys(Boc)-Thr(But)-Phe-OTmse (Stufe 3.7B) und 0,61 g HONB in 30 ml Dimethylformamid gibt man 1,23 g Dicyclohexylcarbodiimid zu. Nach 15 Stunden bei 20° filtriert man ab und giesst das Filtrat in 250 ml Eiswasser. Es wird abfiltriert, getrocknet und durch Säulenchromatographie am Silicagel gereinigt.
DC : Chloroform-Methanol (8 : 2) : Rf 0,85

Stufe 3.7D Bmp(Trt)-Lys(Boc)-Asn-Phe-Phe-(D-Trp)-Lys(Boc)-Thr(But)-Phe-OH

2,50 g Bmp(Trt)-Lys(Boc)-Asn-Phe-Phe-(D-Trp)-Lys(Boc)-Thr(But)-Phe-OTmse (Stufe 3.7C) werden in eine Lösung von 1,48 g Tetraäthylammoniumfluorid in 55 ml Dimethylformamid aufgenommen. Nach 20 Minuten bei 30° wird die Lösung unter Eiskühlung zu 200 ml 0,01-N Salzsäure gegossen, abfiltriert und getrocknet.
DC : Chloroform-Methanol (8 : 2) : Rf 0,42

Stufe 3.8 Bmp(Trt)-Lys(Boc)-Asn-Phe-Phe-(D-Trp)-Lys(Boc)-Thr(But)-Phe-Thr(But)-Tpo-Cys(Trt)-OBut

Zu einer Lösung von 426 mg Bmp(Trt)-Lys(Boc)-Asn-Phe-Phe-(D-Trp)-Lys-(Boc)-Thr(But)-Phe-OH (Stufe 3.7D), 240 mg H-Thr(But)-Tpo-Cys(Trt)-OBut · HCl (Stufe 3.7), 41,8 µl N-Aethylmorpholin und 50 mg HONB in 6 ml Dimethylformamid gibt man 62 mg Dicyclohexylcarbodiimid. Nach 15 Stunden bei 20° wird der Niederschlag abfiltriert, das Filtrat auf die Hälfte eingedampft und mit Wasser ausgefällt. Nach Abnütschen und Trocknen wird die Titelverbindung noch säulenchromatographich an Silicagel gereinigt.
DC : Chloroform-Methanol (8 : 2) : Rf 0,82
Chloroform-Methanol (95 : 5) : Rf 0,10

Stufe 3.9 Bmp-Lys(Boc)-Asn-Phe-Phe-(D-Trp)-Lys(Boc)-Thr(But)-Phe-Thr(But)-Tpo-Cys-OBut (Bmp und Cys durch Bindungsstrich verbunden)

590 mg Bmp(Trt)-Lys(Boc)-Asn-Phe-Phe-(D-Trp)-Lys(Boc)-Thr(But)-Phe-Thr(But)-Tpo-Cys(Trt)-OBut (Stufe 3.8) werden in einem Gemisch von 400 ml Methanol und 10 ml Dimethylformamid gelöst und die gerührte Lösung mit 99,2 ml 0,1-N Jod in Methanol tropfenweise versetzt. Nach weiteren 10 Minuten giesst man 13 ml einer 10 %-igen Lösung von Ascorbinsäure in Wasser zu, engt die farblose Lösung auf ca. 40 ml ein und fällt mit 100 ml Wasser aus. Der Rückstand wird getrocknet und einer Gegenstromverteilung im System Methanol-Puffer (28,6 ml Eisessig, 19,3 g Ammoniumacetat, 1 Liter Wasser)-Chloroform-Tetrachlorkohlenstoff (2 700 : 675 : 900 : 1 575) unterworfen. Nach 500 Verteilungsschritten befindet sich die Substanz in den Elementen 60-84 (K~0,15).

DC : Chloroform-Methanol (8 : 2) : Rf 0,75

Beispiel 4

Bmp-Lys-Asn-Phe-Phe-(D-Trp)-Lys-Thr-Phe-Thr-MeLeu-Cys-OH (MeLeu = N-Methyl-leucin)

118 mg Bmp-Lys(Boc)-Asn-Phe-Phe-(D-Trp)-Lys(Boc)-Thr(But)-Phe-Thr(But)-MeLeu-Cys-OBut werden bei 0° in 60 ml Trifluoressigsäure-Wasser (9 : 1) aufgenommen, nach 30 Minuten bei 30° eingedampft, und der Rückstand mit 30 ml Aether verrieben. Das rohe Trifluoracetat wird im Vakuum getrocknet und der Gegenstromverteilung im System n-Butanol-Wasser Eisessig (4 : 5 : 1) unterworfen. Nach 260 Verteilungsschritten befindet sich das Produkt in den Elementen 35-65 (K~1,95). Die Titelverbindung ist dünnschichtchromatographisch einheitlich.

DC : System 101 : Rf 0,55

Der Ausgangsstoff wird folgendermassen hergestellt:

Stufe 4.1 Z-MeLeu-OMe

Eine Lösung von 25,13 g Z-Leu-OH in 275 ml Tetrahydrofuran-Dimethylformamid (10 : 1) wird mit 50 ml Methyljodid versetzt. Zu dieser Lösung wird unter intensivem Rühren innerhalb 3 Stunden bei 20°-40° portionenweise 14,4 g einer Dispersion von Natriumhydrid zugegeben, das Gemisch zum Rückfluss erhitzt und während 20 Stunden verrührt. Das Reaktionsgemisch wird eingedampft, der Rückstand 3 mal mit je 300 ml Aether versetzt und eingedampft. Danach wird der Rückstand zwischen 250 ml Wasser und 250 ml Aether verteilt, die Aetherphase 2 mal mit je 100 ml Wasser gewaschen und die wässrigen Phasen mit 100 ml Aether nachextrahiert. Vereinigte Aetherphasen werden über Natriumsulfat getrocknet, abfiltriert und eingedampft. Das Produkt wird durch Säulenchromatographie an Silicagel gereinigt.

DC : Chloroform-Aethylacetat (1 : 1) : Rf 0,67
Chloroform-Methanol (1 : 1) : Rf 0,80

Stufe 4.2 Z-MeLeu-OH

23,29 g Z-MeLeu-OMe (Stufe 4.1) werden in 215 ml Dioxan gelöst, mit 72 ml Wasser verdünnt und mit 44 ml 2-N Natriumhydroxid-Lösung versetzt. Nach 10-minutigem Rühren bei 20° wird die Lösung auf 0-5° abgekühlt und mit 21,8 ml 2-N Salzsäure tropfenweise behandelt. Die klare gelbliche Lösung wird eingedampft, mit 150 ml Aethylacetat und 150 ml Wasser versetzt und im Eisbad mit 21,8 ml 2-N Salzsäure tropfenweise behandelt. Die wässrige Phase wird abgetrennt und mit 100 ml Aethylacetat nachextrahiert. Vereinigte organische Phasen werden 3 mal mit je 100 ml Wasser gewaschen, über Natriumsulfat getrocknet und eingedampft. Das Produkt wird aus Cyclohexan umkristallisiert.

F = 74-75°

DC : Chloroform-Aethylacetat (1 : 1) : Rf 0,13
Choroform-Methanol (1 : 1) : Rf 0,54

Stufe 4.3 Z-MeLeu-OTmse

Eine Lösung von 2,79 g Z-MeLeu-OH (Stufe 4.2), 1,61 ml Pyridin und 1,59 ml 2-Trimethylsilyläthanol in 40 ml Acetonitril wird bei −5° innerhalb 30 Minuten portionsweise mit 2,27 g Dicyclohexylcarbodiimid versetzt. Nach 15 Stunden bei 0-5° wird der gebildete Niederschlag abfiltriert. Das Filtrat wird mit 300 ml Aethylacetat verdünnt und 4 mal mit je 25 ml 2-N Salzsäure, 4 mal mit je 50 ml Wasser, 4 mal mit je 50 ml 1-N Natriumhydrogencarbonat-Lösung und 5 mal mit je 50 ml Wasser gewaschen, über Natriumsulfat getrocknet und eingedampft.

DC : Chloroform-Methanol (8 : 2) : Rf 0,80

Stufe 4.4 H-MeLeu-OTmse · Hydrochlorid

Eine Lösung von 3,70 g Z-MeLeu-OTmse (Stufe 4.3) in 40 ml Methanol wird auf 0,37 g Pd-Kohle (10 %-ig) während 15 Minuten hydriert, wobei Ph durch allmähliche Zugabe verdünnter HCl bei 4,5 konstant gehalten wird. Das Reaktionsgemisch wird durch Filtration vom Katalysator befreit und eingedampft. Als Rückstand resultiert praktisch reine Titelverbindung.

DC : Chloroform-Methanol (8 : 2) : Rf 0,71

Stufe 4.5 Bpoc-Thr(But)-MeLeu-OTmse

Eine Lösung von 0,70 g H-MeLeu-OTmse · Hydrochlorid (Stufe 4.4) und 0,316 ml N-Aethylmorpholin in 16 ml Dimethylformamid wird mit 2,43 g [Bpoc-Thr(But)-]$_2$O (Stufe 3.3A) versetzt. Nach 15 Stunden bei 20° wird die Reaktionslösung eingedampft und der Rückstand durch Säulenchromatographie an

Silicagel gereinigt.
DC : Toluol-Aethylacetat (7 : 3) : Rf 0,70

Stufe 4.6 Bpoc-Thr(But)-MeLeu-OH

Eine Lösung von 617 mg Bpoc-Thr(But)-MeLeu-OTmse (Stufe 4.5) in 2 ml Dimethylformamid wird mit einer 0,70-molaren Lösung von Tetraäthylammoniumfluorid in Dimethylsulfoxid versetzt. Nach 1 Stunde bei 20° wird mit 50 ml 0,01-N Salzsäure ausgefällt, abfiltriert und getrocknet.
DC : Toluol-Aethylacetat (7 : 3) : Rf 0,23

Stufe 4.7 Bpoc-Thr(But)-MeLeu-Cys(Trt)-OBut

Eine Lösung von 344 mg Bpoc-Thr(But)-MeLeu-OH (Stufe 4.6), 294 mg H-Cys(Trt)-OBut und 125 mg HONB in 6 ml Dimethylformamid wird mit 157 mg Dicyclohexycarbodiimid versetzt. Nach 15 Stunden bei 20° wird der Niederschlag abfiltriert und das Filtrat eingedampft. Der Rückstand wird durch Säulenchromatographie an Silicagel gereinigt.
DC : Tetrachlorkohlenstoff-Aethylacetat (6 : 4) : Rf 0,70

Stufe 4.8 H-Thr(But)-MeLeu-Cys(Trt)-OBut-Hydrochlorid

Eine Lösung von 350 mg Bpoc-Thr(But)-MeLeu-Cys(Trt)-OBut in 20 ml eines Gemisches von 2,2,2-Trifluoräthanol-Wasser (9 : 1) wird durch allmähliche Zugabe von 0,350 ml 1-N Salzsäure während 30 Minuten bei pH 1,0 gehalten. Das Reaktionsgemisch wird mit Pyridin auf pH 4,3 gestellt, eingedampft und aus tert-Butylalkohol lyophylisiert.
DC : Chloroform-Methanol (8 : 2) : Rf 0,74

Stufe 4,9 Bmp(Trt)-Lys(Boc)-Asn-Phe-Phe-(D-Trp)-Lys(Boc)-Thr(But)-Phe-Thr(But)-MeLeu-Cys(Trt)-OBut

Zu einer Lösung von 486 mg Bmp(Trt)-Lys(Boc)-Asn-Phe-Phe-(D-Trp)-Lys-(Boc)-Thr(But)-Phe-OH (Stufe 3.7D), 275 mg H-Thr(But)-MeLeu-Cys(Trt)-OBut.Hcl (Stufe 4.8), 0,472 ml N-Aethylmorpholin und 56,2 mg HONB in 7 ml Dimethylformamid gibt man 70,6 mg Dicyclohexylcarbodiimid. Nach 15 Stunden bei 20° wird abfiltriert, das Filtrat eingedampft und der Rückstand durch Säulenchromatographie an Silicagel gereinigt.
DC : Chloroform-Methanol (8 : 2) : Rf 0,82
Chloroform-Methanol (95 : 5) : Rf 0,30

Stufe 4.10 Bmp-Lys(Boc)-Asn-Phe-Phe-(D-Trp)-Lys(Boc)-Thr(But)-Phe-Thr(But)-MeLeu-Cys-OBut

382 mg Bmp(Trt)-Lys(Boc)-Asn-Phe-Phe-(D-Trp)-Lys(Boc)-Thr(But)-Phe-Thr(But)-MeLeu-Cys(Trt)-OBut (stufe 4.9) werden in 17 ml Dimethylformamid gelöst und zur gerührten Lösung von 406 mg Jod in 230 ml Methanol innert 2 Minuten zugetropft. Nach weiteren 15 Minuten bei 20° wird eine Lösung von 318 mg Ascorbinsäure in 15 ml Wasser zugesetzt, die farblose Lösung auf ca. 40 ml eingeengt und mit 50 ml Wasser ausgefällt. Der Rückstand wird getrocknet und einer Gegenstromverteilung im System Methanol-Wasser-Chloroform-Tetrachlorkohlenstoff (2 700 : 675 : 900 : 7 575) unterworfen. Nach 380 Verteilungsschritten befindet sich die Substanz in den Elementen 26-45 (K~0,08).
DC : Chloroform-Methanol (8 : 2) : Rf 0,70.

## Beispiel 5

A. Eine Injektionslösung enthaltend 2,0 mg eines gemäss einem der Beispiele 1-4 erhaltenen Peptids, wie

Bmp-Lys-Asn-Phe-Phe-(D-Trp)-Lys-Thr-Phe-Thr-Pro-Cys-OH,

weiterhin in Beispielen 5-11 als « Wirkstoff » bezeichnet, wird folgendermassen erhalten :
Man löst 1,0 mg Eisessig, 0,8 mg Natriumacetat, 8,0 mg Natriumchlorid und 2,0 mg Wirkstoff in 0,7 ml destilliertem Wasser und füllt mit destilliertem Wasser auf 1 ml auf. Die Lösung wird bei 120 °C 20 Minuten lang im Autoklav erhitzt. Das pH nach der Sterilisation ist 4,5.
B. Eine Injektionslösung enthaltend 0,5 mg des Wirkstoffes wird folgendermassen erhalten :
Man löst in 0,7 ml physiologischer Natriumchloridlösung 0,5 mg Wirkstoff und säuert die Lösung mit 0,1-N Salzsäure auf pH 4,0 an. Mit destilliertem Wasser wird auf 1 ml aufgefüllt und sterilfiltriert.

Beispiel 6

A. Eine Gelatine-enthaltende Injektionslösung enthaltend 0,1 mg Wirkstoff wird folgendermassen erhalten :

Eine sterilfiltrierte wässrige Lösung des Wirkstoffes wird unter Erwärmen mit einer sterilen Gelatinelösung, welche als Konservierungsmittel Phenol enthält, unter aseptischen Bedingungen vermischt, sodass 1,0 ml Lösung folgende Zusammensetzung hat :

| | |
|---|---|
| Wirkstoff | 0,1 mg |
| Gelatine | 150,0 mg |
| Phenol | 4,7 mg |
| dest. Wasser bis zu | 1,0 ml |

Die Mischung wird aseptisch in Vials zu 1,0 ml abgefüllt.

B. Eine analoge Injektionslösung enthaltend 0,5 mg des Wirkstoffes erhält man in gleicher Weise wie oben angegeben, indem man eine Mischung der folgenden Zusammensetzung herstellt :

| | |
|---|---|
| Wirkstoff | 0,5 mg |
| Gelatine | 280,0 mg |
| Phenol | 5,0 mg |
| dest. Wasser bis zu | 1,0 ml |

Die Mischung wird aseptisch in Vials zu 1,0 ml abgefüllt.

Beispiel 7

Präparat enthaltend 0,5 mg Wirkstoff als sterile Trockensubstanz zur Injektion wird folgendermassen erhalten : Man löst 0,5 mg Wirkstoff in 1 ml einer wässrigen Lösung von 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in destilliertem Wasser gelöst. Die Lösung wird intramuskulär oder intravenös angewendet.

Beispiel 8

Injektionspräparat enthaltend den Wirkstoff als Polyphosphat-Suspension wird folgendermassen erhalten :

A. Mit 1,0 mg Wirkstoff :

Man mischt eine Lösung von 1,0 mg Wirkstoff und 9,0 mg Natriumchlorid in 0,5 ml destilliertem Wasser mit einer Lösung von 2 mg Natriumpolyphosphat « Calgon N ® » in 0,5 ml destilliertem Wasser. Die erhaltene Suspension hat folgende Zusammensetzung :

| | |
|---|---|
| Wirkstoff | 1,0 mg |
| Natriumpolyphosphat (Calgon N ®) | 2,0 mg |
| Natriumchlorid | 9,0 mg |
| dest. Wasser bis zu | 1,0 ml |

Die Suspension weist ein pH von 6,9 auf. Sie ist für intramuskuläre Anwendung geeignet.

B. Mit 0,5 mg Wirkstoff :

In gleicher Weise wie oben angegeben wird eine Suspension der folgenden Zusammensetzung hergestellt :

| | |
|---|---|
| Wirkstoff | 0,5 mg |
| Natriumpolyphosphat (Calgon 322$^R$) | 1,0 mg |
| Natriumchlorid | 9,0 mg |
| dest. Wasser bis zu | 1,0 ml |

Das pH der Suspension beträgt 5,9.

Beispiel 9

Injektionspräparat enthaltend 0,3 mg Wirkstoff in Form eines öligen Aluminiumstearat-Gels.

Ein 2 %iges Aluminiumstearat-Gel wird auf übliche Art und Weise durch Suspendieren von 1,0 g Aluminiummonostearat in 49,0 g Erdnussöl und anschliessendes Erwärmen auf 130 °C während 10 Min. hergestellt. 15,0 mg Wirkstoff werden mit 0,3 g obigem Aluminiumstearat-Gel suspendiert, homogenisiert

und mit der restlichen Menge Aluminiumstearat-Gel verdünnt. Das so erhaltene Gel hat folgende Zusammensetzung :

| | |
|---|---|
| Wirkstoff | 0,3 mg |
| Aluminiummonostearat | 20,0 mg |
| Erdnussöl bis zu | 1,0 ml |

Die ölige Aluminiumstearat-Gel-Suspension ist für intramuskuläre Anwendung geeignet.

Beispiel 10

Injektionspräparat enthaltend 0,5 mg Wirkstoff als eine Depot-Suspension mit Dextransulfat.

Man löst in 0,4 ml destilliertem Wasser 0,36 mg Essigsäure, 1,9 mg Natriumacetat-trihydrat, 0,8 mg Natriumchlorid und 0,5 mg Wirkstoff und füllt mit destilliertem Wasser auf 0,5 ml auf. Zu dieser Lösung wird unter Rühren 0,5 ml einer 0,1 %igen Lösung von Dextransulfat (Molekulargewicht 500'000) gegeben, womit sich eine homogene Ausfällung bildet. Die erhaltene Suspension hat folgende Zusammensetzung :

| | |
|---|---|
| Wirkstoff | 0,50 mg |
| Dextransulfat MG 500'000 | 0,50 mg |
| Essigsäure 100 % | 0,36 mg |
| Natriumacetat-trihydrat | 1,90 mg |
| Natriumchlorid | 0,80 mg |
| dest. Wasser bis zu | 1,00 ml |

Die wässrige Suspension ist für intramuskuläre und subcutane Injektion geeignet.

Beispiel 11

Nasal-Spray

30 mg feingemahlener Wirkstoff wird in einer Mischung von 75 mg Benzylalkohol und 1,395 g Miglyol 812 suspendiert. Mit dieser Suspension werden Aluminium-Monoblocdosen (Gehalt 10 ml) eingefüllt, mit einem Dosierventil verschlossen und mit 6,0 g Freon 12/114 (40 : 60) unter Stickstoffdruck eingefüllt. Die Aluminiumdose mit einem Füllgewicht von insgesamt 7,5 g enthält 100 Einzeldosen à 0,3 mg Wirkstoff. Die Sprühdose ist mittels des Ventils so eingestellt, dass bei einmaligem Druck eine Einzeldosis versprüht wird.

In gleicher Weise werden Nasensprays hergestellt, welche statt des Miglyols die gleiche Menge Isopropylmyristat oder Isopropylpalmitat oder « Labrafac WL 1 219 » (eine Mischung von Glycerin- und Polyoxyäthylenglycolestern von Fettsäuren mit 8 und 10 Kohlenstoffatomen) enthalten.

**Ansprüche** (für die Vertragsstaaten : BE, CH, DE, FR, GB, IT, LU, NL, SE)

1. Verfahren zur Herstellung von Verbindungen der allgemeinen Formel

Bmp-Lys-X-Phe-Phe-trp-Lys-Thr-Phe-Thr-Y-Cys-OH
3 4 5 6 7 8 9 10 11 12 13 14

worin Bmp für den Desaminocystein-Rest, X für Asn oder His, trp für D-Trp, welches im Benzolring durch ein Halogenatom substituiert sein kann, und Y für den Rest einer sekundären α-Aminosäure mit höchstens 8 Kohlenstoffatomen steht, der entsprechenden Peptidamide, sowie von Säureadditionssalzen und Komplexen aller dieser Verbindungen, dadurch gekennzeichnet, dass man in einer Verbindung der allgemeinen Formel

Bmp(C)-Lys(A)-X-Phe-Phe-trp-Lys(A')-Thr(B)-Phe-Thr(B')-Y-Cys(C')-D (II)

worin X, trp und Y die obgenannten Bedeutungen haben und A und A' unabhängig voneinander je eine ε-Amino-Schutzgruppe oder Wasserstoff, B und B' unabhängig voneinander je eine Hydroxyl-Schutzgruppe oder Wasserstoff, C und C' unabhängig voneinander je eine Mercapto-Schutzgruppe oder Wasserstoff und D eine Carboxyl-Schutzgruppe, die Aminogruppe $NH_2$ oder Hydroxyl bedeuten, in beliebiger Reihenfolge a) vorhandenen Schutzgruppen abspaltet und b) zwischen den Mercaptogruppen beider endständigen Säuren gegebenenfalls unter gleichzeitiger Abspaltung vorhandener Mercapto-Schutzgruppen die Disulfid-Brücke bildet und, wenn erwünscht, einen als Säureadditionssalz resultierenden Endstoff in die entsprechende freie Base oder einen als Base resultierenden Endstoff in ein Säureadditionssalz davon umwandelt und/oder, wenn erwünscht, einen Endstoff in Form eines Komplexes isoliert.

2. Verfahren gemäss Anspruch 1, dadurch gekennzeichnet, dass man die Disulfid-Brücke oxidativ bildet.

3. Ein Somatostatin-analoges Peptid der allgemeinen Formel

Bmp-Lys-X-Phe-Phe-trp-Lys-Thr-Phe-Thr-Y-Cys-OH
3 4 5 6 7 8 9 10 11 12 13 14

worin Bmp für den Desaminocystein-Rest, X für Asn oder His, trp für D-Trp, welches im Benzolring durch ein Halogenatom substituiert sein kann, und Y für den Rest einer sekundären α-Aminosäure mit höchstens 8 Kohlenstoffatomen steht, und ein entsprechendes Peptidamid, sowie Säureadditionssalze und Komplexe dieser Verbindungen.

4. Eine Verbindung der Formel I, worin X für Asn, trp für D-Trp oder 5-Fluor-D-Trp und Y für Pro, den Rest des 4-Thiaprolins oder N-Methyl-Leu steht.

5. [Desamino-Cys$^3$-D-Trp$^8$-Pro$^{13}$]-somatostatin(3-14).

6. [Desamino-Cys$^3$-(5-Fluor-D-Trp)$^8$-Pro$^{13}$]-somatostatin(3-14).

7. [Desamino-Cys$^3$-D-Trp$^8$-Tpo$^{13}$]-somatostatin(3-14).

8. [Desamino-Cys$^3$-D-Trp$^8$-MeLeu$^{13}$]-somatostatin(3-14).

9. Pharmazeutisch verwendbare Säureadditionssalze und Komplexe von Verbindungen gemäss einem der Ansprüche 3-8.

10. Pharmazeutische Präparate enthaltend mindestens eine der Verbindungen gemäss einem der Ansprüche 3-9.

11. Verwendung der Verbindungen gemäss einem der Ansprüche 3-9 zur Herstellung eines Arzneimittels, dadurch gekennzeichnet, dass man mindestens eine dieser Verbindungen mit mindestens einem pharmazeutisch verwendbarem Hilfs- oder Trägermaterial vermischt.

12. Verbindung gemäss einem der Ansprüche 3-9 als Antidiabeticum.

**Ansprüche** (für den Vertragsstaat AT)

1. Verfahren zur Herstellung von Verbindungen der allgemeinen Formel

Bmp-Lys-X-Phe-Phe-trp-Lys-Thr-Phe-Thr-Y-Cys-OH
3 4 5 6 7 8 9 10 11 12 13 14

worin Bmp für den Desaminocystein-Rest, X für Asn oder His, trp für D-Trp, welches im Benzolring durch ein Halogenatom substituiert sein kann, und Y für den Rest einer sekundären α-Aminosäure mit höchstens 8 Kohlenstoffatomen steht, der entsprechenden Peptidamide, sowie von Säureadditionssalzen und Komplexen aller dieser Verbindungen, dadurch gekennzeichnet, dass man in einer Verbindung der allgemeinen Formel

Bmp(C)-Lys(A)-X-Phe-Phe-trp-Lys(A')-Thr(B)-Phe-Thr(B')-Y-Cys(C')-D (II)

worin X, trp und Y die obgenannten Bedeutungen haben und A und A' unabhängig voneinander je eine ε-Amino-Schutzgruppe oder Wasserstoff, B und B' unabhängig voneinander je eine Hydroxyl-Schutzgruppe oder Wasserstoff, C und C' unabhängig voneinander je eine Mercapto-Schutzgruppe oder Wasserstoff und D eine Carboxyl-Schutzgruppe, die Aminogruppe $NH_2$ oder Hydroxyl bedeuten, in beliebiger Reihenfolge a) vorhandene Schutzgruppen abspaltet und b) zwischen den Mercaptogruppen beider endständigen Säuren gegebenenfalls unter gleichzeitiger Abspaltung vorhandener Mercapto-Schutzgruppen die Disulfid-Brücke bildet und, wenn erwünscht, einen als Säureadditionssalz resultierenden Endstoff in die entsprechende freie Base oder einen als Base resultierenden Endstoff in ein Säureadditionssalz davon umwandelt und/oder, wenn erwünscht, einen Endstoff in Form eines Komplexes isoliert.

2. Verfahren gemäss Anspruch 1, dadurch gekennzeichnet, dass man eine Verbindung der Formel I, worin X für Asn, trp für D-Trp oder 5-Fluor-D-Trp und Y für Pro, den Rest des 4-thiaprolins oder N-Methyl-Leu steht, herstellt.

3. Verfahren gemäss Anspruch 1, dadurch gekennzeichnet, dass man [Desamino-Cys$^3$-D-Trp$^8$-Pro$^{13}$]-somatostatin(3-14) herstellt.

4. Verfahren gemäss Anspruch 1, dadurch gekennzeichnet, dass man [Desamino-Cys$^3$-(5-Fluor-D-Trp)$^8$-Pro$^{13}$]-somatostatin(3-14) herstellt.

5. Verfahren gemäss Anspruch 1, dadurch gekennzeichnet, dass man [Desamino-Cys$^3$-D-Trp$^8$-

$Tpo^{13}$]-somatostatin(3-14) herstellt.

6. Verfahren gemäss Anspruch 1, dadurch gekennzeichnet, dass man [Desamino-$Cys^3$-D-$Trp^8$-$MeLeu^{13}$]-somatostatin(3-14) herstellt.

7. Verfahren gemäss Anspruch 1, dadurch gekennzeichnet, dass man pharmazeutisch verwendbare Säureadditionssalze oder Komplexe von Verbindungen gemäss einem der Ansprüche 2-6 herstellt.

8. Verfahren gemäss einem der Ansprüche 1-7, dadurch gekennzeichnet, dass man die Disulfid-Brücke oxidativ bildet.

9. Verfahren zur Herstellung von pharmazeutischen Präparaten enthaltend mindestens eine der gemäss einem der Ansprüche 1-7 erhältlichen Verbindungen, dadurch gekennzeichnet, dass man mindestens eine dieser Verbindungen mit mindestens einem pharmazeutisch verwendbarem Hilfs- oder Trägermaterial vermischt.

10. Verfahren zur Herstellung von pharmazeutischen Präparaten enthaltend mindestens eine der gemäss Anspruch 8 erhältlichen Verbindungen, dadurch gekennzeichnet, dass man mindestens eine dieser Verbindungen mit mindestens einem pharmazeutisch verwendbarem Hilfs- oder Trägermaterial vermischt.

11. Verwendung der Verbindungen gemäss einem der Ansprüche 1-7 zur Herstellung eines Arzneimittels, dadurch gekennzeichnet, dass man mindestens eine dieser Verbindungen mit mindestens einem pharmazeutisch verwendbarem Hilfs- oder Trägermaterial vermischt.

12. Verwendung der Verbindungen gemäss Anspruch 8 zur Herstellung eines Arzneimittels, dadurch gekennzeichnet, dass man mindestens eine dieser Verbindungen mit mindestens einem pharmazeutisch verwendbarem Hilfs- oder Trägermaterial vermischt.

**Claims** (for the Contracting States : BE, CH, DE, FR, GB, IT, LU, NL, SE)

1. A process for the manufacture of compounds of the general formula

Bmp-Lys-X-Phe-Phe-trp-Lys-Thr-Phe-Thr-Y-Cys-OH
3 4 5 6 7 8 9 10 11 12 13 14

wherein Bmp represents the desaminocysteine radical, X represents Asn or His, trp represents D-Trp which may be substituted in the benzene ring by a halogen atom, and Y represents the radical of a secondary α-amino acid having a maximum of 8 carbon atoms, and the corresponding peptide amides and also acid addition salts and complexes of all these compounds, characterised in that, in a compound of the general formula

Bmp(C)-Lys(A)-X-Phe-Phe-trp-Lys(A')-Thr(B)-Phe-Thr(B')-Y-Cys(C')-D (II)

in which X, trp and Y have the meanings given above and each of A and A' independently of the other represents an ε-amino-protective group or hydrogen, each of B and B' independently of the other represents a hydroxyl-protective group or hydrogen, each of C and C' independently of the other represents a mercapto-protective group or hydrogen and D represents a carboxyl-protective group, the amino group $NH_2$ or hydroxyl, in any order, a) any protective groups present are split off and b) the disulphide bridge is formed between the mercapto groups of both terminal acids optionally while simultaneously splitting off any mercapto-protective groups present and, if desired, an end product formed as an acid addition salt is converted into the corresponding free base or an end product formed as a base is converted into an acid addition salt thereof and/or, if desired, an end product is isolated in the form of a complex.

2. A process according to claim 1, characterised in that the disulphide bridge is formed by means of oxidation.

3. A somatostatin-analogous peptide of the general formula

Bmp-Lys-X-Phe-Phe-trp-Lys-Thr-Phe-Thr-Y-Cys-OH
3 4 5 6 7 8 9 10 11 12 13 14

in which Bmp represents the desaminocysteine radical, X represents Asn or His, trp represents D-Trp which may be substituted in the benzene ring by a halogen atom, and Y represents the radical of a secondary α-amino acid having a maximum of 8 carbon atoms, and a corrèsponding peptide amide, and also acid addition salts and complexes of these compounds.

4. A compound of the formula I, wherein X represents Asn, trp represents D-trp or 5-fluoro-D-Trp and Y represents Pro, the radical of 4-thiaproline or N-methyl-Leu.

5. [Desamino-$Cys^3$-D-$Trp^8$-$Pro^{13}$]-somatostatin(3-14).

6. [Desamino-$Cys^3$-(5-fluoro-D-Trp$)^8$-$Pro^{13}$]-somatostatin(3-14).

7. [Desamino-Cys$^3$-D-Trp$^8$-Tpo$^{13}$]-somatostatin(3-14).

8. [Desamino-Cys$^3$-D-Trp$^8$-MeLeu$^{13}$]-somatostatin(3-14).

9. A pharmaceutically acceptable acid addition salt or complex of a compound according to any one of claims 3 to 8.

10. A pharmaceutical preparation containing at least one compound according to any one of claims 3 to 9.

11. Use of a compound according to any one of claims 3 to 9 for the manufacture of a medicament, characterised in that at least one such compound is mixed with at least one pharmaceutically acceptable excipient or carrier.

12. A compound according to one of claims 3 to 9 as an antidiabetic.

**Claims** (for the Contracting State AT)

1. A process for the manufacture of compounds of the general formula

Bmp-Lys-X-Phe-Phe-trp-Lys-Thr-Phe-Thr-Y-Cys-OH
3 4 5 6 7 8 9 10 11 12 13 14

wherein Bmp represents the desaminocysteine radical, X represents Asn or His, trp represents D-Trp which may be substituted in the benzene ring by a halogen atom, and Y represents the radical of a secondary $\alpha$-amino acid having a maximum of 8 carbon atoms, and the corresponding peptide amides and also acid addition salts and complexes of all these compounds, characterised in that, in a compound of the general formula

Bmp(C)-Lys(A)-X-Phe-Phe-trp-Lys(A')-Thr(B)-Phe-Thr(B')-Y-Cys(C')-D (II)

in which X, trp and Y have the meanings given above and each of A and A' independently of the other represents an $\varepsilon$-amino-protective group or hydrogen, each of B and B' independently of the other represents a hydroxyl-protective group or hydrogen, each of C and C' independently of the other represents a mercapto-protective group or hydrogen and D represents a carboxyl-protective group, the amino group $NH_2$ or hydroxyl, in any order, a) any protective groups present are split off and b) the disulphide bridge is formed between the mercapto groups of both terminal acids optionally while simultaneously splitting off any mercapto-protective groups present and, if desired, an end product formed as an acid addition salt is converted into the corresponding free base or an end product formed as a base is converted into an acid addition salt thereof and/or, if desired, an end product is isolated in the form of a complex.

2. A process according to claim 1 for the manufacture of a compound of the formula I, wherein X represents Asn, trp represents D-trp or 5-fluoro-D-Trp and Y represents Pro, the radical of 4-thiaproline or N-methyl-Leu.

3. A process according to claim 1 for the manufacture of [desamino-Cys$^3$-D-Trp$^8$-Pro$^{13}$]-somatostatin(3-14).

4. A process according to claim 1 for the manufacture of [desamino-Cys$^3$-(5-fluoro-D-Trp)$^8$-Pro$^{13}$]-somatostatin(3-14).

5. A process according to claim 1 for the manufacture of [desamino-Cys$^3$-D-Trp$^8$-Tpo$^{13}$]-somatostatin(3-14).

6. A process according to claim 1 for the manufacture of [desamino-Cys$^3$-D-Trp$^8$-MeLeu$^{13}$]-somatostatin(3-14).

7. A process according to claim 1 for the manufacture of a pharmaceutically acceptable acid addition salt or complex of a compound according to any one of claims 2 to 6.

8. A process according to any one of claims 1 to 7, characterised in that the disulphide bridge is formed by oxidation.

9. A process for the manufacture of a pharmaceutical preparation containing at least one of the compounds obtainable according to any one of claims 1 to 7, characterised in that at least one such compound is mixed with at least one pharmaceutically acceptable excipient or carrier.

10. A process for the manufacture of a pharmaceutical preparation containing at least one compound obtainable according to claim 8, characterised in that at least one such compound is mixed with a pharmaceutically acceptable excipient or carrier.

11. Use of a compound according to any one of claims 1 to 7 for the manufacture of a medicament, characterised in that at least one such compound is mixed with at least one pharmaceutically acceptable excipient or carrier.

12. Use of a compound according to claim 8 for the manufacture of a medicament, characterised in that at least one such compound is mixed with at least one pharmaceutically acceptable excipient or carrier.

**Revendications** (pour les Etats contractants : BE, CH, DE, FR, GB, IT, LU, NL, SE)

1. Procédé de préparation de composés de formule générale

Bmp-Lys-X-Phe-Phe-trp-Lys-Thr-Phe-Thr-Y-Cys-OH
3 4 5 6 7 8 9 10 11 12 13 14

où Bmp représente le radical désaminocystéine, X Asn ou His, trp D-Trp, qui peut être substitué dans le noyau benzène par un atome d'halogène, et Y le radical d'un acide α-aminé secondaire ayant au plus 8 atomes de carbone, des peptidamides correspondants, ainsi que des sels d'addition d'acides et des complexes de tous ces composés, caractérisé en ce que dans un composé de formule générale

Bmp(C)-Lys(A)-X-Phe-Phe-trp-Lys(A')-Thr(B)-Phe-Thr(B')-Y-Cys-(C')-D (II)

où X, trp et Y ont les significations données ci-dessus et A et A' représentent indépendamment l'un de l'autre chacun un groupe protecteur d'ε-amino ou un hydrogène, B et B' représentent chacun indépendamment l'un de l'autre un groupe protecteur d'hydroxyle ou un hydrogène, C et C' représentent chacun indépendamment l'un de l'autre un groupe protecteur de mercapto ou un hydrogène et D représente un groupe protecteur de carboxyle, le groupe amino $NH_2$ ou un hydroxyle, dans un ordre quelconque a) on sépare les groupes protecteurs présents et b) on forme le pont disulfure entre les groupes mercapto des deux acides en position finale, éventuellement en séparant simultanément les groupes protecteurs de mercapto présents, et, si on le désire, on transforme un produit final obtenu sous forme de sel d'addition d'acide en la base libre correspondante ou un produit final obtenu sous forme de base en un de ses sels d'addition d'acides, et/ou, si on le désire, on isole un produit final sous forme de complexe.

2. Procédé selon la revendication 1, caractérisé en ce qu'on forme le pont disulfure de manière oxydative.

3. Peptide analogue de somatostatine de formule générale

Bmp-Lys-X-Phe-Phe-trp-Lys-Thr-Phe-Thr-Y-Cys-OH
3 4 5 6 7 8 9 10 11 12 13 14

où Bmp représente le radical désaminocystéine, X Asn ou His, trp D-Trp, qui dans le noyau benzène peut être substitué par un atome d'halogène, et Y le radical d'un acide α-aminé secondaire ayant au plus 8 atomes de carbone, et un peptidamide correspondant, ainsi que les sels d'addition d'acides et complexes de ces composés.

4. Composé de formule I où X représente Asn, trp représente D-trp ou 5-fluoro-D-Trp et Y représente Pro, le radical de la 4-thiaproline ou la N-méthyl-Leu.

5. [Désamino-$Cys^3$-D-$Trp^8$-$Pro^{13}$]-somatostatine(3-14).

6. [Désamino-$Cys^3$-(5-fluoro-D-Trp)$^8$-$Pro^{13}$]-somatostatine(3-14).

7. [Désamino-$Cys^3$-D-$Trp^8$-$Tpo^{13}$]-somatostatine(3-14).

8. [Désamino-$Cys^3$-D-$Trp^8$-$MeLeu^{13}$]-somatostatine(3-14).

9. Sels d'addition d'acides et complexes pharmaceutiquement acceptables de composés selon l'une des revendications 3-8.

10. Préparations pharmaceutiques contenant au moins un des composés selon l'une des revendications 3-9.

11. Application des composés selon l'une des revendications 3-9 à la préparation d'un médicament, caractérisée en ce qu'on mélange au moins l'un de ces composés avec au moins un additif ou support pharmaceutiquement utilisable.

12. Composé selon l'une des revendications 3-9 comme agent anti-diabétique.

**Revendications** (pour l'Etat contractant AT)

1. Procédé de préparation de composés de formule générale

Bmp-Lys-X-Phe-Phe-trp-Lys-Thr-Phe-Thr-Y-Cys-OH
3 4 5 6 7 8 9 10 11 12 13 14

où Bmp représente le radical désaminocystéine, X représente Asn ou His, trp représente D-Trp, qui peut être substitué dans le noyau benzène par un atome d'halogène et Y représente le radical d'un acide α-

aminé secondaire ayant au plus 8 atomes de carbone, des peptidamides correspondants, ainsi que des sels d'addition d'acides et complexes de tous ces composés, caractérisé en ce que dans un composé de formule générale

Bmp(C)-Lys(A)-X-Phe-Phe-trp-Lys(A')-Thr(B)-Phe-Thr(B')-Y-Cys(C')-D (II)

où X, trp et Y ont les significations données ci-dessus et où A et A' représentent indépendamment l'un de l'autre chacun un groupe protecteur d'ε-amino ou un hydrogène, B et B' représentent chacun indépendamment l'un de l'autre un groupe protecteur d'hydroxyle ou un hydrogène, C et C' représentent indépendamment l'un de l'autre chacun un groupe protecteur de mercapto ou un hydrogène et D représente un groupe protecteur de carboxyle, le groupe amino $NH_2$ ou un hydroxyle, dans un ordre quelconque, a) on sépare les groupes protecteurs présents et b) on forme le pont disulfure entre les groupes mercapto des deux acides en position finale, éventuellement en séparant simultanément les groupes protecteurs de mercapto présents, et, si on le désire, on transforme un produit final obtenu sous forme de sel d'addition d'acides en la base libre correspondante ou un produit final obtenu sous la forme d'une base en un de ses sels d'addition d'acides, et/ou, si on le désire, on isole un produit final sous forme d'un complexe.

2. Procédé selon la revendication 1, caractérisé en ce qu'on prépare un composé de formule I où X représente Asn, trp représente D-Trp ou 5-fluoro-D-Trp et Y représente Pro, le radical de la 4-thiaproline ou la N-méthyl-Leu.

3. Procédé selon la revendication 1, caractérisé en ce qu'on prépare la [Désamino-$Cys^3$-D-$Trp^8$-$Pro^{13}$]-somatostatine(3-14).

4. Procédé selon la revendication 1, caractérisé en ce qu'on prépare la [Désamino-$Cys^3$-(5-fluoro-D-$Trp)^8$-$Pro^{13}$]-somatostatine(3-14).

5. Procédé selon la revendication 1, caractérisé en ce qu'on prépare la [Désamino-$Cys^3$-D-$Trp^8$-$Tpo^{13}$]-somatostatine(3-14).

6. Procédé selon la revendication 1, caractérisé en ce qu'on prépare la [Désamino-$Cys^3$-D-$Trp^8$-$MeLeu^{13}$]-somatostatine(3-14).

7. Procédé selon la revendication 1, caractérisé en ce qu'on prépare les sels d'addition d'acides ou complexes pharmaceutiquement acceptables de composés selon l'une des revendications 2-6.

8. Procédé selon l'une des revendications 1-7, caractérisé en ce qu'on forme le pont disulfure de façon oxydative.

9. Procédé de préparation de préparations pharmaceutiques contenant au moins l'un des composés obtenus selon l'une des revendications 1-7, caractérisé en ce qu'on mélange au moins l'un de ces composés avec au moins un additif ou support pharmaceutiquement acceptable.

10. Procédé de préparation de préparations pharmaceutiques contenant au moins l'un des composés obtenus selon la revendication 8, caractérisé en ce qu'on mélange au moins un de ces composés avec au moins un additif ou support pharmaceutiquement acceptable.

11. Application des composés selon l'une des revendications 1-7 à la préparation d'un médicament, caractérisée en ce qu'on mélange au moins un de ces composés avec au moins un additif ou support pharmaceutiquement acceptable.

12. Application des composés selon la revendication 8 à la préparation d'un médicament, caractérisée en ce qu'on mélange au moins un de ces composés avec au moins un additif ou support pharmaceutiquement acceptable.